# FASCICULE DE BREVET EUROPEEN

(11) **EP 1 986 616 B1**
(45) Date de publication et mention de la délivrance du brevet: **15.08.2018**
(21) Numéro de dépôt: 07712232.3
(22) Date de dépôt: 16.02.2007
(51) Int. Cl.: A61K 9/48, A61K 9/50, A61K 9/16, A61K 9/52, A61K 9/32, A61K 9/26

(54) **FORMES PHARMACEUTIQUES MULTIMICROPARTICULAIRES QUI RESISTENT A LA DECHARGE IMMEDIATE DU PRINCIPE ACTIF EN PRESENCE D'ALCOOL**
GEGEN SOFORTIGE FREISETZUNG DES WIRKSTOFFS IN GEGENWART VON ALKOHOL WIDERSTANDSFÄHIGE MIKROTEILCHENFÖRMIGE PHARMAZEUTISCHE FORMULIERUNGEN
MICROPARTICULATE PHARMACEUTICAL FORMS RESISTANT TO IMMEDIATE RELEASE OF THE ACTIVE PRINCIPLE IN THE PRESENCE OF ALCOHOL

(30) Priorité: 16.02.2006 FR 0650566
(43) Date de publication de la demande: 05.11.2008
(73) Titulaire: Flamel Ireland Limited, Dublin 15 (IE)
(72) Inventeur: GUIMBERTEAU, Florence, 33450 Montussan (FR); DARGELAS, Frédéric, 69007 Lyon (FR)
(74) Mandataire: Cabinet Plasseraud
(86) Numéro de dépôt international: PCT/EP2007/051528
(87) Numéro de publication internationale: WO 2007/093642

(56) Documents cités:
- EP-A- 1 557 179
- EP-A- 1 721 604
- EP-A1- 0 635 261
- EP-A1- 1 044 682
- WO-A-98/32426
- WO-A-99/20255
- WO-A-99/55313
- WO-A-03/013525
- WO-A-2004/096177
- WO-A-2004/103350
- WO-A-2005/011666
- WO-A-2005/084639
- WO-A-2006/106344
- WO-A1-2007/054378
- WO-A2-2004/056337
- WO-A2-2005/102287
- WO-A2-2006/038226
- WO-A2-2006/125819
- FR-A- 2 872 044
- FR-A1- 2 850 576
- US-A- 3 784 390
- US-A- 5 472 712
- US-A- 6 103 261
- US-A- 6 129 933
- US-A1- 2003 180 361
- US-B2- 6 569 463
- US-B2- 6 656 507
- ROBERT J. MEYER, AJAZ S. HUSSAIN: "Mitigating the Risks of Ethanol Induced Dose Dumping from Oral Sustained/Controlled Release Dosage Forms"[Online] octobre 2005 (2005-10), pages 1-4, XP002409099 FDA's ACPS Meeting Extrait de l'Internet: URL:www.fda.gov/ohrms/dockets/ac/05/briefi ng/2005-4187B1_01_08-Alcohol-Induced.pdf> [extrait le 2006-11-23]
- AJAZ S. HUSSAIN: "Preventing Alcohol Induced Dose Dumping is a Desired Product Design Feature"[Online] 26 octobre 2005 (2005-10-26), pages 1-13, XP002409100 ACPS Meeting Extrait de l'Internet: URL:http://www.fda.gov/ohrms/dockets/ac/05 /slides/2005-4187S2_02_Hussain_files/frame .htm> [extrait le 2006-11-23]
- U.S. FOOD AND DRUG ADMINISTRATION: "Palladone (Hydromorphone Hydrochloride Extended-Release) Capsules Questions and Answers"[Online] 13 juillet 2005 (2005-07-13), pages 1-3, XP002409101 Extrait de l'Internet: URL:http://www.fda.gov/cder/drug/infopage/ palladone/palladoneQA.htm> [extrait le 2006-11-23]
- PURDUE PHARMA: "Medication Guide: Palladone"[Online] 24 septembre 2004 (2004-09-24), pages 1-6, XP002409102 USA Extrait de l'Internet: URL:http://www.joinwaypharm.com/pages/prod ucts-e-3.htm> [extrait le 2006-11-23]

## Description

### DOMAINE DE L'INVENTION

Le domaine de la présente invention est celui des formes pharmaceutiques ou diététiques à libération modifiée de principes actifs médicamenteux (PA) destinés à une administration par la voie orale.

La présente invention concerne des formes destinées à l'administration per os, contenant au moins un PA, et capables de maintenir une libération modifiée du PA dans une solution alcoolique, c'est-à-dire non sujettes à une décharge de la dose en présence d'alcool. De préférence l'invention concerne des formes pharmaceutiques à libération modifiée dont le profil de libération n'est pas affecté de manière significative en solution alcoolique.

La présente invention concerne plus particulièrement les formes du type visé au paragraphe précédent et comprenant une pluralité de microparticules réservoirs.

La présente invention concerne encore plus particulièrement les formes pharmaceutiques pour lesquelles l'ingestion d'alcool lors de l'administration est déconseillée.

L'invention concerne également un procédé de préparation des formes pharmaceutiques définies ci-dessus.

### CONTEXTE DE L'INVENTION

L'intérêt des formes pharmaceutiques à libération modifiée pour l'administration d'un médicament est bien connu. Elles permettent en particulier de mieux assurer la couverture du besoin thérapeutique, car la concentration plasmatique utile en PA peut être maintenue plus longtemps que dans le cas des formes à libération instantanée. De plus elles permettent d'éviter, ou de limiter, l'importance et le nombre des pics de concentration plasmatique excessive en PA, ce qui diminue la toxicité du médicament et ses effets secondaires. Par ailleurs, ces systèmes permettent, par leur durée d'action accrue, de limiter le nombre de prises quotidiennes, ce qui diminue la contrainte pour le patient et améliore l'observance du traitement.

Il a ainsi été recherché des systèmes permettant de prolonger l'action d'un médicament, et les références concernant cet objectif sont nombreuses. On consultera à cet égard l'ouvrage de Buri, Puisieux, Doelker et Benoît : Formes Pharmaceutiques Nouvelles, Lavoisier 1985, p. 175-227.

Des formes à libération modifiées (MR, pour *Modified Release* en anglais) ont été mises au point, en particulier pour des PA à fenêtre thérapeutique étroite, c'est-à-dire dont les doses efficaces sont proches de celles auxquelles peuvent se manifester des effets indésirables, afin d'écrêter le pic plasmatique (Cmax), l'objectif étant de maintenir des concentrations plasmatiques pendant une durée prolongée et qui restent en deçà des valeurs où des effets indésirables sont à redouter.

On a également développé de telles formes pour permettre une imprégnation de l'organisme en PA plus stable et continue, sans que le sujet ait besoin de multiplier les prises. Il existe ainsi des formes contenant, dans une unité de prise, la quantité de PA nécessaire pour 24 h de traitement, cette forme étant bien sûr destinée à être administrée une seule fois par jour.

Parmi les formes pharmaceutiques à libération modifiée on peut distinguer les systèmes où la libération du PA est contrôlée par un enrobage entourant le PA, ces systèmes étant également dénommés systèmes réservoirs. Dans un autre groupe, celui des systèmes matriciels, le PA, intimement dispersé dans une matrice, par exemple à base de polymère, est libéré du comprimé par diffusion et érosion.

De nombreux travaux ont été mis en oeuvre pour s'assurer que la libération du PA est effectivement contrôlée, afin d'éviter le surdosage massif qui résulterait d'une libération accidentellement immédiate de la quantité de PA prévue pour une libération prolongée. Ce contrôle est extrêmement important en pratique, puisque ce sont le plus souvent des produits actifs à fenêtre thérapeutique étroite qui bénéficient de la technique de libération modifiée. Dans ce cas, cette libération immédiate accidentelle *(dose dumping* en anglais, soit littéralement 'décharge de la dose') aurait des effets exactement opposés à ceux que la technique mise en oeuvre s'efforçait d'atteindre.

Une décharge de la dose peut se produire par exemple dans le cas d'un comprimé monolithique matriciel que le patient mâcherait avant de déglutir, court-circuitant une étape de lent délitement dans l'estomac. Une façon avantageuse d'éviter le risque lié à la mastication consiste à préparer une forme microparticulaire, chaque microparticule possédant les propriétés de libération modifiée.

Le recours à des formes multi(micro)particulaires limite le risque de libération massive, et permet de diminuer la variabilité inter- et intra-individuelle liée à la vidange gastrique.

La demande PCT WO-A-96/11675 décrit des microcapsules à libération modifiée pour l'administration per os de principes actifs médicamenteux et/ou nutritionnels (PA), dont la taille est inférieure ou égale à 1000 µm. Ces microcapsules sont constituées par des particules enrobées par un matériau d'enrobage constitué par un mélange d'un polymère filmogène (éthylcellulose), d'un agent plastifiant hydrophobe (huile de ricin), d'un agent tensioactif ou lubrifiant (stéarate de magnésium) et d'un polymère azoté (polyvinylpyrrolidone : povidone, PVP). Ces microcapsules sont également caractérisées par leur aptitude à séjourner longtemps (au moins 5 h) dans l'intestin grêle et à permettre, lors de ce séjour, l'absorption du PA sur une période supérieure au temps de transit naturel dans l'intestin grêle.

La demande PCT WO-A-03/030878 décrit une forme pharmaceutique orale multimicrocapsulaire dans laquelle la libération du PA est régie par un double mécanisme de déclenchement de la libération : "temps déclenchant" et "pH déclenchant". Cette forme est constituée de microcapsules (200 à 600 µm) comprenant un coeur contenant le PA et recouvert d'un enrobage (maximum 40% en poids), ce dernier comprenant un polymère A hydrophile porteur de fonctions ionisées à pH neutre (Eudragit® L) et un composé B hydrophobe (cire végétale dont le point de fusion est égal à 40-90 °C), avec B/A compris entre 0,2 et 1,5.

L'enrobage des microcapsules peut comprendre, outre les constituants essentiels A et B, d'autres ingrédients classiques, tels que notamment :
- des colorants ;
- des plastifiants comme par exemple le dibutylsébaçate ;
- des composés hydrophiles comme par exemple la cellulose et ses dérivés ou la polyvinylpyrrolidone et ses dérivés ;
- et leurs mélanges.

Ces exemples illustrent les efforts entrepris pour éviter une défaillance des différents systèmes à libération modifiée du (ou des) PA.

Cependant, malgré ces efforts, il est récemment apparu qu'une libération trop rapide de la masse du PA peut se produire lors de l'ingestion concomitante de la forme pharmaceutique MR et d'alcool.

Ainsi aux USA en octobre 2005, la Food and Drug Administration a émis l'idée selon laquelle une étude de la résistance des formes MR à une décharge de la dose (dose dumping) potentiellement induite par l'alcool mériterait d'être menée pour certains médicaments. Hussain et al. 2005 (URLs : www.fda.gov/ohrms/dockets/ac/05/briefing/2005-4187B1_01_08-Alcohollnduced.pdf; www.fda.gov/horms/dockets/ac/05/slides/2005-4187S2_02_Hussain_files/frame.htm) soulève le problème technique d'identifier des nouvelles formes pharmaceutiques à libération modifiée, non sensibles à l'alcool. Hussain propose une forme pharmaceutique de type granule matriciel de cire comprenant un enrobage (à base du composé « XXX » non divulgué), la résistance à l'alcool étant liée au caractère « insoluble » de la matrice du fait de sa compression et de l'enrobage de type gélifiant.

En effet, des études récentes ont montré que la présence d'alcool peut accélérer la libération d'un PA contenu dans une forme pharmaceutique MR. Cet effet de l'alcool peut, en première analyse, s'expliquer par une altération du système à libération modifiée ou par une modification de la solubilité du PA en présence d'une quantité significative d'alcool. Cette situation a d'autant plus de probabilité de se rencontrer - et les conséquences risquent d'être d'autant plus lourdes - que la quantité de boisson alcoolisée ingérée est importante, que le degré d'alcool de la boisson est élevé et que le sujet est à jeun. En effet dans ce dernier cas, l'estomac contiendra essentiellement la boisson ingérée mêlée à une faible quantité de suc gastrique.

Donc, en pratique, l'ingestion d'alcool parallèlement à l'administration d'une forme pharmaceutique MR peut conduire à la libération accélérée et potentiellement dangereuse du PA chez le patient. En fonction du type de PA, cette libération accélérée du PA, au mieux, rend la forme pharmaceutique MR totalement inefficace, et au pire, compromet le pronostic vital du patient.

Cette accélération néfaste de la libération peut conduire à une perte d'activité du médicament : il en sera ainsi par exemple des inhibiteurs de pompes à protons, dont la libération trop précoce en milieu gastrique acide conduira à leur dégradation, et donc à l'inefficacité du traitement.

Inversement, plus redoutable est le cas de certains tranquillisants, antidépresseurs ou celui des analgésiques opiacés, où c'est le pronostic vital qui sera en jeu en raison de la gravité des effets secondaires consécutifs à un surdosage.

Un ensemble particulier de médicaments pour lesquels une libération massive du PA serait particulièrement néfaste est celui des produits pour lesquelles il existe une interaction pharmacologique défavorable avec l'alcool, une incompatibilité, ou un renforcement des effets secondaires :
- ainsi par exemple les médicaments du groupe des analgésiques opiacés ont comme effet indésirable de pouvoir induire une dépression respiratoire ; celle-ci peut être aggravée par la prise concomitante d'alcool en raison des fausses routes et des pneumopathies de déglutition provoquées classiquement par l'abus d'alcool ;
- également des médicaments très utilisés comme les tranquillisants et
- les antidépresseurs ont des effets sur le système nerveux central (perte de la vigilance, risques de somnolence) qui sont accentués par la prise simultanée d'alcool ;
- on peut encore citer les interactions de l'alcool avec les antihistaminiques (potentialisation de l'effet sédatif, somnolence et perte d'attention, étourdissements), et
- les anti-inflammatoires non stéroïdiens ou AINS (potentialisation du risque de saignements digestifs).

Dans le cas des formes monolithiques matricielles, la décharge accidentelle de la dose entraine des concentrations de PA très élevées au niveau digestif où se trouve la forme, ce qui peut y déterminer des lésions.

Le problème de la décharge de la dose en présence d'alcool n'a pas été résolu de manière satisfaisante à ce jour, en particulier dans le cas des formes multimicroparticulaires. Il existe notamment un besoin d'une forme pharmaceutique multimicroparticulaire à libération modifiée pour l'administration de PA par voie orale, capable de maintenir la libération modifiée du PA dans une solution alcoolique, c'est-à-dire dont le profil de libération du PA n'est pas accéléré au risque d'engager le pronostic vital d'un patient, et, de préférence, dont le profil de libération du PA n'est pas affecté de manière significative en solution alcoolique.

La demande de brevet WO03/013525 décrit une forme pharmaceutique orale comprenant (i) un agoniste d'opioïde, (ii) un antagoniste d'opioïde sous forme libérable et (iii) un antagoniste d'opioïde sous une forme piégé lorsque la forme pharmaceutique est administrée intacte (donc non accessible directement). La demande de brevet EP1557179 décrit également des formes pharmaceutiques solides comprenant un agoniste d'opioïde et un antagoniste d'opioïde. La demande de brevet EP1044682 décrit un procédé de fabrication de gélules à base d'HPMC. La demande de brevet WO2006/106344 décrit des gélules remplies avec un principe actif et agent modificateur et résistantes au mésusage. Ces gélules peuvent être enrobées par un enrobage entérique. La demande de brevet WO2004/056337 décrit une forme pharmaceutique orale comprenant un coeur à libération contrôlée et une gélule de gélatine à libération immédiate de principe actif et encapsulant le coeur à libération contrôlée. La demande de brevet WO2006/125819 décrit des médicaments oraux multimicroparticulaires, permettant la prévention du mésusage du principe actif et comprenant des microparticules enrobées à libération modifiée de principe actif. La demande de brevet WO2007/054378 porte également sur des formes pharmaceutiques orales multimicroparticulaires permettant d'éviter le mésusage du principe actif qu'elle contient.

### OBJECTIFS DE L'INVENTION

Un objectif essentiel de la présente invention est de proposer une forme pharmaceutique multimicroparticulaire à libération modifiée d'au moins un principe actif médicamenteux ou diététique (PA), destinée à l'administration par voie orale, permettant d'éviter ou de limiter une décharge de la dose (*dose dumping* en anglais) induite par la consommation d'alcool lors de l'administration de cette forme pharmaceutique, ce qui permet d'assurer une plus grande sécurité thérapeutique et une meilleure efficacité.

Un autre objectif essentiel de la présente invention est de proposer une forme pharmaceutique multimicroparticulaire à libération modifiée d'au moins un PA, destinée à l'administration par voie orale, pour laquelle la libération du PA n'est pas significativement affectée par la présence d'alcool.

Les microparticules selon l'invention sont sous forme de gélules

Un autre objectif essentiel de la présente invention est de proposer une forme pharmaceutique multimicroparticulaire à libération modifiée d'au moins un PA, destinée à l'administration par voie orale, pour laquelle le profil de libération *in vitro* du PA dans les milieux de dissolution classiquement utilisés et exempts d'éthanol est similaire au profil obtenu dans ces mêmes milieux auxquels de l'éthanol a été ajouté.

Un autre objectif essentiel de la présente invention est de proposer une forme pharmaceutique multimicroparticulaire à libération modifiée d'au moins un PA, destinée à l'administration par voie orale, présentant un profil de libération *in vitro* en présence d'éthanol, lequel profil n'engage pas le pronostic vital d'un patient.

Un autre objectif essentiel de la présente invention est de fournir une forme pharmaceutique multimicroparticulaire à libération modifiée d'au moins un PA, destinée à l'administration par voie orale, perfectionné par rapport à celles décrites dans les demandes internationales WO-A-96/11675 et WO-A-03/03878, en particulier au regard du comportement en solution alcoolique.

Un autre objectif essentiel de la présente invention est de proposer un procédé d'obtention d'une forme pharmaceutique multimicroparticulaire à libération modifiée d'au moins un PA, destinée à l'administration par voie orale et dont le profil de libération du PA *in vitro* n'est pas affecté de manière significative en solution alcoolique, ou au moins dont la libération n'est pas accélérée au risque d'engager le pronostic vital du patient.

### Définitions

Au sens du présent exposé de l'invention :
- l'abréviation « PA » désigne aussi bien un seul principe actif qu'un mélange de plusieurs principes actifs. Le PA peut être sous forme libre ou sous forme de sel, d'ester, d'hydrate, de solvate, de polymorphe, d'isomères ou d'autres formes pharmaceutiquement acceptables ;
- l'alcool ingéré peut provenir de différentes boissons ou breuvages alcoolisés telles que bière, vin, cocktails, spiritueux ou leurs mélanges ;
- *in vitro,* le terme « alcool » représente l'éthanol et les termes « solution alcoolique » ou « milieu alcoolique » représente une solution aqueuse d'éthanol ;
- le terme « hypromellose » représente l'hydroxypropylméthylcellulose ou HPMC ;
- « microparticules réservoir » désigne des microparticules comprenant du PA et individuellement enrobées par au moins un enrobage permettant la libération modifiée du PA ;
- « microparticule » désigne indifféremment des microparticules réservoir et/ou des microparticules comprenant du PA non nécessairement enrobées ;
- les profils de dissolution *in vitro* sont réalisés selon les indications de la pharmacopée européenne (5^{ème} édition, § 2.9.3) où les milieux de dissolution classiquement utilisés sont décrits. Pour simuler le milieu gastrique d'un sujet ayant absorbé une forte quantité d'alcool, le milieu de dissolution est modifié par addition d'éthanol (q.s.p. 20 % à 40 % en volume) ;
- l'abréviation « MR » signifie : à libération modifiée ;
- le terme « libération modifiée » signifie que la libération du PA *in vitro* est telle que 75 % du PA est libéré en un temps supérieur à 0,75 h et, de préférence, supérieur à 1 h, et plus préférentiellement supérieur à 1,5 h. Une forme pharmaceutique à libération modifiée peut, par exemple, comprendre une phase à libération immédiate et une phase à libération lente. La libération modifiée peut être notamment une libération prolongée et/ou retardée. Des formes pharmaceutiques à libération modifiée sont bien connues dans ce domaine ; voir par exemple Remington : The science and practice ofpharmacy, 19ème édition, Mack publishing Co. Pennsylvanie, USA ;
- « libération immédiate » signifie que la libération n'est pas de type libération modifiée et désigne la libération par une forme à libération immédiate de la plus grande partie du PA en un temps relativement bref, par exemple au moins 75 % du PA sont libérés en 0,75 h, de préférence en 30 min ;
- les formes pharmaceutiques orales multimicroparticulaires selon l'invention sont constituées de nombreuses microparticules dont la taille est inférieure au millimètre. Les diamètres de microparticules dont il est question dans le présent exposé sont, sauf indication contraire, des diamètres moyens en volume. Ces formes multimicroparticulaires peuvent être transformées en formes pharmaceutiques orales monolithiques telles que gélules, ;
- la similarité entre deux profils de dissolution est évaluée à l'aide du facteur de similarité f₂ tel qu'il est défini dans le document "Qualité des produits à libération modifiée" de l'Agence européenne pour l'évaluation du médicament, document référencé CPMP/QWP/604/96 (Annexe 3). Une valeur de f₂ comprise entre 50 et 100 indique que les deux profils de dissolution sont similaires ;
- par « décharge de la dose » (*dose dumping*)*,* on entend une libération immédiate et non voulue de la dose après ingestion *per os.*

### BREVE DESCRIPTION

Il est du mérite des inventeurs d'avoir trouvé une formulation qui permet de supprimer ou de diminuer les modifications des profils de libération du PA observées en solution alcoolique.

Les présents inventeurs ont élaboré des formes pharmaceutiques MR présentant une résistance à une décharge de la dose induite par l'alcool. Cette propriété avantageuse peut notamment être mise en évidence dans des conditions reproduisant les caractéristiques physico-chimiques attendues *in vivo.* L'alcoolisme périodique ou *"binge drinking",* forme d'alcoolisme se caractérisant par des accès de forte consommation, typiquement en fin de semaine, alternant avec de longues périodes d'abstinence ou de modération, est devenu dans certains milieux une activité sociale de plus en plus répandue. Et parallèlement a augmenté le risque que représente une libération accidentelle de la dose de PA contenue dans une forme pharmaceutique MR chez un sujet qui aurait également ingéré une forte quantité d'alcool.

Les inventeurs ont effectué des travaux d'étude de la sensibilité de diverses formes pharmaceutiques MR en présence d'alcool. L'approche qui a été retenue pour mesurer la résistance des formes pharmaceutiques MR à une décharge de la dose induite par l'alcool consiste à modifier les tests classiques de dissolution des formes pharmaceutiques MR en introduisant de l'éthanol dans le milieu de dissolution, par exemple à une concentration de 20 % ou de 40 % (v/v). L'ordre de grandeur du volume final est de 50 à 900 mL.

Pour un certain nombre de formes pharmaceutiques MR, on observe que la co-administration de ladite forme avec des breuvages alcoolisés conduirait à une accélération non voulue de la libération du ou des PA. Pour résoudre ce problème, la présente invention a pour objet une nouvelle forme pharmaceutique multimicroparticulaire à libération modifiée d'au moins un PA médicamenteux, destinée à l'administration par voie orale, caractérisée en ce qu'elle est capable de maintenir la libération modifiée du PA dans une solution alcoolique, et de préférence en ce que le profil de libération n'est pas affecté de manière significative en solution alcoolique.

Plus précisément, la présente invention vise une forme pharmaceutique ou diététique orale comprenant des microparticules de type réservoir à libération prolongée d'au moins un principe actif (PA) et au moins un agent D qui est un composé pharmaceutiquement acceptable dont la vitesse ou la capacité à s'hydrater ou à se solvater est supérieure en milieu aqueux exempt d'alcool qu'en solution alcoolique, l'agent D étant en mélange avec les microparticules de type réservoir et représentant de 2 à 30% p/p de la masse totale du mélange
ladite forme pharmaceutique orale résistant à la décharge immédiate de la dose de PA en présence d'alcool
ladite forme pharmaceutique ou diététique orale se présentant sous la forme d'une gélule, ledit agent D étant choisi dans le groupe constitué par :
- les carboxyalkylcelluloses réticulées : les carboxyméthylcelluloses réticulées (e.g. croscarmellose de sodium),
- les (hydroxy)(alkyl)celluloses (e.g. hydroxypropylcellulose, hydroxypropyl-méthylcellulose),
- les carboxyalkylcelluloses (e.g. carboxyméthylcellulose) et leurs sels,
- les polysaccharides,
- les protéines,
- et leurs mélanges.

De préférence, la forme pharmaceutique orale selon l'invention, qui comprend des microparticules de type réservoir à libération modifiée d'au moins un PA aussi bien dans les milieux de dissolution aqueux que dans les solutions alcooliques, est caractérisée en ce que le temps de libération de 50 % du PA en solution alcoolique :
- n'est pas diminué de plus de 3 fois par rapport au temps de libération de 50 % du PA mesuré en milieu aqueux exempt d'alcool ;
- de préférence n'est pas diminué de plus de 2 fois par rapport au temps de libération de 50 % du PA mesuré en milieu aqueux exempt d'alcool ;
- de préférence n'est pas diminué de plus de 1,5 fois par rapport au temps de libération de 50 % du PA mesuré en milieu aqueux exempt d'alcool ;
- de préférence est similaire à celui mesuré en milieu aqueux, d'après le facteur de similarité f₂ défini ci-dessus ;
- voire le temps de libération de 50 % du PA en solution alcoolique est supérieur au temps de libération de 50 % du PA en milieu aqueux exempt d'alcool.

Cette forme pharmaceutique selon l'invention comprend des microparticules de type réservoir et au moins un agent D, qui est un composé pharmaceutiquement acceptable et dont la vitesse ou la capacité à s'hydrater ou à se solvater est supérieure en milieu aqueux exempt d'alcool qu'en solution alcoolique. Les microparticules réservoir ont, de préférence, un diamètre moyen inférieur à 2000 µm, plus préférentiellement compris entre 50 et 800 µm, et plus préférentiellement encore compris entre 100 et 600 µm. En outre, les microparticules réservoirs sont individuellement constituées par un coeur comprenant le PA et enrobé par un enrobage comprenant :
- au moins un polymère A insoluble dans les liquides du tube digestif ;
- au moins un agent plastifiant B ;
- éventuellement au moins un agent tensioactif C.

### BREVE DESCRIPTION DES FIGURES

- Figure 1 :: Représentation schématique de la structure d'une microparticule enrobée.
- Figure 2 :: Représentation schématique de la structure d'une microparticule enrobée.
- Figure 3 :: Représentation schématique de la structure d'un pellet ou d'un granule comprenant des microparticules et de l'agent D comme liant.
- Figure 4 :: Représentation schématique d'un comprimé enrobé contenant des microparticules.
- Figure 5 :: Représentation schématique d'une gélule revêtue par un revêtement à base d'agent D, la gélule contenant des microparticules.
- Figure 6 :: Dissolution des gélules d'aciclovir préparées en exemple 1.
- Figure 7 :: Dissolution des gélules de metformine préparées à l'exemple 2.
- Figure 8 :: Dissolution des gélules d'aciclovir préparées en exemple 3.
- Figure 9 :: Dissolution des gélules de metformine préparées à l'exemple 4.
- Figure 10 :: Comportement de l'amidon glycolate de sodium (Primojel® / Avebe) dans l'eau (Fig. 10A) et dans une solution alcoolique (Fig. 10B) après 15 min de contact.
- Figure 11 :: Comportement de la gomme guar (Grindsted® Guar / Danisco) dans l'eau (Fig. 11A) et dans une solution alcoolique (Fig. 11B) après 15 min de contact.
- Figure 12 :: Comportement de l'hydroxypropylméthyl cellulose (Methocel® E5 / Dow) dans l'eau (Fig. 12A) et dans une solution alcoolique (Fig. 12B) après 30 min de contact.
- Figure 13 :: Dissolution des gélules de metformine préparées à l'exemple 6.

### DESCRIPTION DETAILLEE

Il est décrit une forme pharmaceutique ou diététique orale qui comprend des microparticules de type réservoir et permet la libération modifiée du PA aussi bien dans les milieux de dissolution aqueux que dans les solutions alcooliques. Cette forme selon la présente description est multimicroparticulaire, c'est-à-dire qu'elle comprend entre autres des microparticules réservoirs avec un coeur comprenant le PA enrobé ou pelliculé par un enrobage. Ce coeur de PA, ou microparticule de PA, peut être :
- du PA brut (pur) sous forme pulvérulente, et/ou
- un granulé matriciel de PA mélangé à différents autres ingrédients, et/ou
- un granulé supporté, tel qu'un support neutre, par exemple en cellulose ou en sucre, recouvert d'au moins une couche comportant du PA.

Dans le cas d'un granulé matriciel, la matrice contient le PA et éventuellement d'autres excipients pharmaceutiquement acceptables, tels que des agents liants, des tensioactifs, des désintégrants, des charges, des agents contrôlant ou modifiant le pH (tampons).

Dans le cas d'un granulé supporté, le support neutre peut être composé de saccharose et/ou de dextrose et/ou de lactose, et/ou de mélange saccharose/amidon. Le support neutre peut également être une microsphère de cellulose ou tout autre particule d'excipient pharmaceutiquement acceptable. Avantageusement, le support neutre a un diamètre moyen compris entre 1 et 800 µm et de préférence compris entre 20 et 500 µm.

La couche active peut éventuellement comporter, outre le (ou les) PA, un ou plusieurs excipients pharmaceutiquement acceptables, tels que des agents liants, des tensioactifs, des désintégrants, des charges, des agents contrôlant ou modifiant le pH (tampons).

La forme selon la présente description peut comprendre des microparticules de PA autres que des microparticules réservoirs. Il pourrait s'agir, par exemple, de microparticules à libération immédiate de PA. Ces dernières peuvent être par exemple des microparticules de PA non enrobées, du même type que celles utiles dans la préparation des microparticules réservoirs selon la présente description, et comprenant un ou plusieurs PA.

En outre, l'ensemble des microparticules (microparticules réservoirs et/ou microparticules non enrobées) constituant la forme selon la présente description peut être constitué de différentes populations de microparticules, ces populations différant entre elles au moins par la nature du (ou des) PA contenus dans ces microparticules et/ou par la composition de l'enrobage et/ou l'épaisseur de l'enrobage.

Selon une première forme de réalisation, au moins une partie des microparticules à libération modifiée de PA comporte chacune une microparticule de PA, enrobée par au moins un enrobage permettant la libération modifiée du PA.

De préférence, la microparticule de PA est un granulé comprenant le (les) PA et un ou plusieurs excipients pharmaceutiquement acceptables.

Selon une deuxième forme de réalisation, au moins une partie des microparticules à libération modifiée de PA comporte chacune un support neutre, au moins une couche active comprenant le (ou les) PA et enrobant le support neutre, et au moins un enrobage permettant la libération modifiée du PA.

Comme cela a été rappelé ci-dessus, les microparticules réservoirs sont individuellement constituées par un coeur comprenant le PA et enrobé par un enrobage. L'enrobage régit la libération modifiée du PA. Il comprend :
- au moins un polymère A insoluble dans les liquides du tube digestif ;
- au moins un agent plastifiant B ;
- éventuellement au moins un agent tensioactif C.

L'enrobage des microparticules réservoirs contient un polymère A insoluble dans les liquides du tube digestif à raison de 70 % à 95 %, de préférence de 75 % à 95 %, et, plus préférentiellement encore, de 80 % à 95 % de la masse de l'enrobage hors agent D. Le polymère A est sélectionné, de préférence, dans le groupe de produits suivants :
- les dérivés non hydrosolubles de la cellulose,
- les dérivés de (co)polymères (méth)acryliques,
- et leurs mélanges.

Plus préférentiellement, le polymère A est sélectionné dans le groupe de produits suivants : l'éthylcellulose, l'acétate butyrate de cellulose, l'acétate de cellulose, les copolymères ammonio-méthacrylates type A et type B (Eudragit® RS, Eudragit® RL, Eudragit® RS PO, Eudragit® PL PO), les esters d'acides poly(méth)acryliques (Eudragit® NE 30D) et leurs mélanges ; l'éthylcellulose et/ou l'acétate de cellulose étant particulièrement préférés.

L'agent plastifiant B est présent dans l'enrobage des microparticules réservoirs à raison de 1 % à 30 % p/p, de préférence, de 2 % à 25 % p/p, et, plus préférentiellement encore, de 5 % à 20 % en masse de l'enrobage hors agent D. L'agent plastifiant B est sélectionné notamment dans le groupe de produits suivants :
- le glycérol et ses esters, de préférence dans le sous-groupe suivant : glycérides acétylés, glycéryl-mono-stéarate, glycéryl-triacétate, glycéryl-tributyrate,
- les phtalates, de préférence dans le sous-groupe suivant : dibutylphtalate, diéthylphtalate, diméthylphtalate, dioctylphtalate,
- les citrates, de préférence dans le sous-groupe suivant : acétyltributylcitrate, acétyltriéthylcitrate, tributylcitrate, triéthylcitrate,
- les sébaçates, de préférence dans le sous-groupe suivant : diéthylsébaçate, dibutylsébaçate,
- les adipates,
- les azélates,
- les benzoates,
- le chlorobutanol,
- les polyéthylène glycols,
- les huiles végétales,
- les fumarates, de préférence le diéthylfumarate,
- les malates, de préférence le diéthylmalate,
- les oxalates, de préférence le diéthyloxalate,
- les succinates ; de préférence le dibutylsuccinate,
- les butyrates,
- les esters de l'alcool cétylique,
- les malonates, de préférence le diéthylmalonate,
- l'huile de ricin (celle-ci étant particulièrement préférée),
- et leurs mélanges.

L'agent tensioactif C est présent dans l'enrobage des microparticules réservoirs à raison de 0 à 30 % p/p, de préférence de 0 à 20 % p/p, et, plus préférentiellement encore, de 5 à 15 % de la masse de l'enrobage hors agent D. L'agent tensioactif C est de préférence sélectionné dans le groupe de produits suivants :
- les sels alcalins ou alcalinoterreux des acides gras, le sodium dodécyl sulfate et le docusate de sodium étant préférés,
- les huiles polyoxyéthylénées, de préférence l'huile de ricin hydrogénée polyoxyéthylénée,
- les copolymères polyoxyéthylène-polyoxypropylène,
- les esters de sorbitan polyoxyéthylénés,
- les dérivés de l'huile de ricin polyoxyéthylénés,
- les stéarates, de préférence de calcium, de magnésium, d'aluminium ou de zinc,
- les polysorbates,
- les stéarylfumarates, de préférence de sodium,
- le béhénate de glycérol,
- le chlorure de benzalkonium
- le bromure d'acétyltriméthyl ammonium,
- et leurs mélanges.

L'enrobage mono- ou multicouche peut comprendre divers autres adjuvants additionnels utilisés classiquement dans le domaine de l'enrobage. Il peut s'agir, par exemple, de pigments, de colorants, de charges, d'agents antimousses, etc.

Suivant une modalité particulière de réalisation de la présente description, l'enrobage régissant la libération modifiée du PA par les microparticules réservoirs est constitué d'une seule couche ou une seule pellicule d'enrobage. Cela simplifie leur préparation et limite le taux d'enrobage.

Avantageusement, l'enrobage présente une résistance mécanique suffisante pour éviter son déchirement et/ou son éclatement dans l'organisme et ce jusqu'à la fin de la libération du PA. Cette aptitude de l'enrobage à conserver son intégrité physique même après élution complète du PA s'observe notamment pour des épaisseurs d'enrobage comprises entre 2 µm et 100 µm, soit des taux d'enrobage (masse d'enrobage hors agent D sur masse totale de la microparticule) compris entre 3 et 85%.

Il est important de noter que la fonctionnalité de résistance à l'alcool n'est pas acquise au détriment des autres spécifications exigées pour une forme pharmaceutique à libération modifiée. En particulier, la forme pharmaceutique selon la présente description peut être adaptée à un grand nombre de PA présentant des solubilités dans l'eau très variées, comprises par exemple entre quelques centièmes de milligrammes par litre et quelques centaines de grammes par litre.

Par ailleurs, la forme pharmaceutique selon la présente description permet d'ajuster la libération du PA sur des durées très variées, par exemple comprises entre 1 h et 30 h, de préférences comprises entre 2 h et 16 h. Il est à la portée de l'homme du métier d'ajuster le temps de libération en faisant varier notamment la composition et/ou l'épaisseur de l'enrobage, et/ou la taille moyenne des microparticules.

L'agent D est un composé pharmaceutiquement acceptable dont la vitesse ou la capacité à s'hydrater ou à se solvater est supérieure en milieu aqueux exempt d'alcool qu'en solution alcoolique. Il peut s'agir :
- d'un composé à vitesse de solubilisation plus élevée dans l'eau qu'en solution alcoolique ;
- d'un composé soluble dans l'eau et insoluble en solution alcoolique ; ou
- d'un composé, insoluble dans l'eau ou en solution alcoolique, qui gonfle plus, ou plus vite, dans l'eau qu'en solution alcoolique.

De préférence, l'agent D est choisi dans le groupe de produits suivants :
- les carboxyalkylcelluloses réticulées : les carboxyméthylcelluloses réticulées (par ex. croscarmellose de sodium),
- les polyalkylènes oxydes (par ex. polyéthylène oxyde ou polypropylène oxyde),
- les (hydroxy)(alkyl)celluloses (par ex. hydroxypropylcellulose, hypromellose [ou HPMC]),
- les carboxyalkylcelluloses (par ex. carboxyméthylcellulose) et leurs sels,
- les celluloses (poudre ou microcristalline),
- la polacriline de potassium,
- les polysaccharides, par exemple :
   - les amidons natifs (par ex. de maïs, blé ou pomme de terre) ou modifiés (par ex. avec du glycolate de sodium),
   - les alginates et leurs sels, tel que l'alginate de sodium,
   - les gommes guar,
   - les carraghénanes,
   - les pullulanes,
   - les pectines,
   - les chitosanes et leurs dérivés,
   - et leurs mélanges,
- les protéines, par exemple :
   - la gélatine,
   - les albumines,
   - la caséine,
   - les lactoglobulines,
   - et leurs mélanges,
- les argiles telles que la bentonite, la laponite,
- et leurs mélanges.

De manière plus préférée encore, l'agent D est choisi dans le groupe de produits suivants :
- les hydroxyalkylcelluloses (par ex. hydroxypropylcellulose, hypromellose [ou HPMC] ),
- les gommes guar,
- les carraghénanes,
- les pullulanes,
- et leurs mélanges.

L'agent D peut être incorporé de différentes façons, éventuellement combinées entre elles, dans la forme pharmaceutique selon la présente description. Il peut être :
- un des constituants du coeur de PA (ou microparticule non enrobée de PA), soit :
   - dans le support neutre des microparticules et/ou
   - dans la couche contenant le PA, déposée sur le support neutre des microparticules et/ou
   - dans le granulé contenant le PA ; et/ou
- un des constituants de l'enrobage des microparticules ; et/ou
- en mélange avec les microparticules ; et/ou
- un des constituants extérieurs d'une forme monolithique (par ex. constituant d'une gélule, revêtement d'un comprimé ou d'une gélule).

Suivant un premier mode de réalisation de la présente description, l'agent D est présent dans le coeur de PA, ou microparticule non enrobée de PA. De préférence, l'agent D est présent dans le coeur des microparticules à raison de 5 à 70 %, de préférence de 15 % à 60 % de la masse totale du coeur de PA.

Suivant un deuxième mode de réalisation de la présente description, l'agent D est compris dans l'enrobage des microparticules. Dans ce cas, l'agent D peut constituer à lui seul une couche d'enrobage interne ou externe à l'enrobage contrôlant la diffusion. Il peut également être mélangé aux constituants A, B et éventuellement C de l'enrobage qui régit la libération modifiée du PA. De préférence, l'agent D est présent dans l'enrobage à raison de 3 à 30 %, de préférence de 10 % à 20 %, de la masse totale de l'enrobage. De préférence, on choisit les composés suivants : le polymère A est l'éthylcellulose, l'agent plastifiant B est l'huile de ricin, l'agent tensioactif est le polysorbate, et l'agent D est choisi parmi la gomme guar, l'hypromellose [ou HPMC], la carboxyméthylcellulose de sodium, le pullulane, le glycolate d'amidon, et leurs mélanges.

Suivant un troisième mode de réalisation, l'agent D est inclus dans la phase liante de granules ou de pellets ou encore de comprimés incluant les microparticules. Les granules, pellets ou comprimés sont obtenus par les techniques connues de l'homme de l'art comme par exemple la granulation, l'extrusion ou la compression. L'agent D est présent en mélange avec les microparticules, à raison de 2 à 30 % p/p, de préférence de 5 % à 25 % p/p, et plus préférentiellement encore de 5% à 20 % p/p, de la masse totale du mélange.

Suivant un quatrième mode de réalisation, l'agent D est l'un des composants du matériau constituant la gélule qui contient les microparticules. Par exemple, la gélule se présente sous la forme d'une gélule à base d'un agent D, de préférence à base de pullulane, d'hypromellose [ou HPMC] ou de leur mélange.

Suivant un cinquième mode de réalisation, l'agent D est compris dans un revêtement déposé sur la gélule contenant les microparticules ou sur le comprimé contenant les microparticules. Par exemple, la gélule est à base de gélatine, et le revêtement contient de la carboxyméthylcellulose de sodium comme agent D, de préférence à raison de 25 % p/p de carboxyméthylcellulose de sodium par rapport à la masse des gélules vides.

Dans le cas des quatrième et cinquième modes, on pourra déposer sur la gélule ou le comprimé une couche de finition.

S'agissant de l'agent D, les cinq modes de réalisation peuvent être combinés entre eux ; de plus il est possible d'incorporer différents agents D pour chacun des modes de réalisation.

De préférence la forme selon la présente description est constituée par une ou plusieurs mêmes unités galéniques (par ex. comprimé, gélule ou sachet) contenant chacune les microparticules.

La forme selon la présente description peut également se présenter sous la forme d'une suspension orale multidose, reconstituée à partir de poudre et d'eau avant administration.

La forme selon la présente description peut également se présenter sous la forme d'une gélule renfermant un comprimé, ce comprimé contenant des microparticules réservoirs de PA ; le comprimé peut contenir un ou plusieurs agents D, et la gélule peut-être enrobée par un ou plusieurs agents D.

Avantageusement, la forme contenant les microparticules à libération modifiée de PA comprend également des excipients pharmaceutiquement acceptables, classiques, et utiles par exemple pour présenter les microparticules sous forme de comprimé. Ces excipients peuvent être notamment :
- des agents de compression, comme la cellulose microcristalline ou le mannitol,
- des colorants,
- des désintégrants,
- des agents d'écoulement comme le talc, la silice colloïdale,
- des lubrifiants comme par exemple le béhénate de glycérol, les stéarates,
- des arômes,
- des conservateurs,
- et leurs mélanges.

La forme pharmaceutique finale, sous forme de comprimé ou de gélule, peut être enrobée selon les techniques et formules connues de l'homme de l'art pour améliorer sa présentation : couleur, aspect, masquage de goût, etc.

Les nouvelles formes pharmaceutiques à base de PA selon la présente description sont originales dans leur structure, leur présentation et leur composition et sont administrables *per os,* notamment à dose journalière unique.

Il peut être intéressant de mélanger dans une même gélule, un même comprimé ou une même poudre pour suspension buvable, au moins deux types de microparticules à cinétiques de libération du PA différentes, par exemple à libération immédiate et à libération modifiée. Il peut aussi être intéressant de mélanger deux (ou plusieurs) types de microparticules, contenant chacun un PA différent, libéré selon un profil de libération qui lui est propre.

Ainsi, la présente description vise notamment une forme pharmaceutique multimicroparticulaire, caractérisée en ce qu'elle contient une pluralité de populations de microparticules, lesdites populations différant entre elles au moins par la nature du PA contenu et/ou par la composition de l'enrobage et/ou par l'épaisseur de l'enrobage et/ou par la localisation de l'agent D.

La présente description vise également une forme pharmaceutique multimicroparticulaire, comprenant au moins deux types de microparticules à cinétiques de libération du PA différentes, par exemple à libération immédiate et à libération modifiée ou encore à libération modifiée selon des cinétiques de libération différentes.

La présente description vise aussi une forme pharmaceutique multimicroparticulaire, comprenant en outre un mélange de plusieurs PA, chacun d'entre eux étant contenu dans des microparticules présentant des cinétiques de libération identiques ou différentes.

Sans vouloir être limitatif, il doit être néanmoins souligné que la forme pharmaceutique selon la présente description est particulièrement intéressante en ce qu'elle peut se présenter sous forme de dose unique orale journalière comprenant de 100 (cent) à 500.000 microparticules réservoirs contenant du PA.

Par ailleurs la présente description vise l'utilisation des microparticules telles que définies ci-dessus pour la préparation de formes orales multimicroparticulaires, pharmaceutiques ou diététiques, de préférence sous forme de comprimés, de poudre pour suspension buvable ou de gélules.

Enfin, la présente description vise également un traitement thérapeutique amélioré, consistant essentiellement à administrer une forme pharmaceutique plus sûre en ce qui concerne le risque de décharge de la dose (*dose dumping*) en présence d'alcool.

Selon un autre de ses aspects, la présente description concerne également les microparticules *per se* telles que définies ci-dessus.

La présente description a également pour objet les procédés pour l'obtention des formes pharmaceutiques selon la présente description telles que définies ci-dessus, lesdits procédés se décomposant en plusieurs étapes consistant essentiellement à :
a) préparer des coeurs (microparticules non enrobées) de PA par :
   - extrusion/sphéronisation de PA avec éventuellement un ou plusieurs agent(s) D ou excipient(s) pharmaceutiquement acceptables, et/ou ;
   - granulation humide de PA avec éventuellement un ou plusieurs agent(s) D ou excipient(s) pharmaceutiquement acceptables, et/ou ;
   - compactage de PA avec éventuellement un ou plusieurs agent(s) D ou excipient(s) pharmaceutiquement acceptables, et/ou ;
   - pulvérisation de PA, avec éventuellement un ou plusieurs agent(s) D ou excipient(s) pharmaceutiquement acceptables, en dispersion ou en solution dans un solvant aqueux ou organique sur un support neutre ou des particules d'agent D, et/ou ;
   - tamisage de poudre ou cristaux de PA ;
b) préparer des microparticules réservoirs de PA par :
   - pulvérisation en lit d'air fluidisé d'une solution ou dispersion contenant un ou plusieurs composés A, B et éventuellement un ou plusieurs composés C et/ou D sur les microparticules de PA ; les microparticules de PA peuvent avoir été au préalable enrobées par un ou plusieurs agents D ; les microparticules de PA enrobées peuvent éventuellement être enrobées par un ou plusieurs agents D ;
c) préparer la forme finale du médicament par :
   - granulation et/ou extrusion/sphéronisation des microparticules réservoir de PA avec un agent D pour mise en gélule ou sachet ; ou
   - mélange de microparticules réservoir de PA avec éventuellement un ou plusieurs agent(s) D et des excipients pharmaceutiquement acceptables pour obtention d'un comprimé ; ce comprimé peut éventuellement être enrobé en turbine d'enrobage par une ou plusieurs couches contenant l'agent D et/ou des excipients pharmaceutiquement acceptables ; ou
   - mise en gélule des microparticules réservoirs de PA ; les gélules peuvent éventuellement être enrobées en turbine ou lit d'air fluidisé par un ou plusieurs agent(s) D et/ou des excipients pharmaceutiquement acceptables ; ou
   - mise en sachet des microparticules réservoirs de PA avec éventuellement un ou plusieurs agent(s) D et/ou des excipients pharmaceutiquement acceptables ; ou
   - mise en gélule de comprimés contenant des microparticules réservoirs de PA, le comprimé contenant un ou plusieurs agents D et les gélules pouvant être enrobées par un ou plusieurs agents D.

Il s'agit là de méthodologies générales intéressantes, qui permettent de produire les formes de la présente description d'une manière simple et économique.

La présente description peut être mise en oeuvre indépendamment de la solubilité du PA dans l'eau. Quatre classes de PA sont définies en fonction de leur solubilité, d'après le *Biopharmaceutics Classification System* de la U.S. Food and Drug Administration (Amidon G.L. et al. "A theoretical basis for a biopharmaceutics drug classification: the corrélation of in vivo drug product dissolution and in vivo bioavailability", Pharmaceutical Research, 1995, vol. 12: 413-420). Des PA appartenant à ces différentes classes peuvent être utilisés selon la présente description.

Le PA contenu dans les microparticules enrobées selon la présente description est avantageusement choisi parmi au moins l'une des familles de substances actives suivantes : les agents de traitement de l'abus d'alcool, les agents de traitement de la maladie d'Alzheimer, les anesthésiques, les agents de traitement de l'acromégalie, les analgésiques, les antiasthmatiques, les agents de traitement des allergies, les anticancéreux, les anti-inflammatoires, les anticoagulants et antithrombotiques, les anti-convulsivants, les antiépileptiques, les antidiabétiques, les antiémétiques, les antiglaucomes, les antihistaminiques, les anti-infectieux, les antiparkinsoniens, les anti-cholinergiques, les antitussifs, les inhibiteurs de l'anhydrase carbonique, les agents cardiovasculaires : hypolipémiants, anti-arythmiques, vasodilatateurs, antiangineux, anti-hypertenseurs, vasoprotecteurs et inhibiteurs de cholinestérase, les agents de traitement des désordres du système nerveux central, les stimulants du système nerveux central, les contraceptifs, les promoteurs de fécondité, les inducteurs et inhibiteurs du travail utérin, les agents de traitement de la mucoviscidose, les agonistes des récepteurs de la dopamine, les agents de traitement de l'endométriose, les agents de traitement des dysfonctionnements érectiles, les agents de traitement de la fertilité, les agents de traitements des troubles gastro-intestinaux, les immunomodulateurs et les immunosuppresseurs, les agents de traitement des troubles de la mémoire, les antimigraineux, les myorelaxants, les analogues de nucléosides, les agents de traitement de l'ostéoporose, les parasympathomimétiques, les prostaglandines, les agents psychothérapeutiques : sédatifs, hypnotiques, tranquillisants, neuroleptiques, anxiolytiques, psychostimulants et antidépresseurs, les agents de traitement dermatologiques, les stéroïdes et les hormones, les amphétamines, les anorexigènes, les anti-douleurs non analgésiques, les antiépileptiques, les barbituriques, les benzodiazépines, les hypnotiques, les laxatifs, les psychotropes.

Des agents de traitement de l'abus d'alcool sont par exemple : chlorazépate, chlordiazépoxyde, diazépam, disulfiram, hydroxyzine, naltrexone, et leurs sels, leurs esters, leurs hydrates, leurs polymorphes et leurs isomères.

Des anesthésiques sont par exemple : lidocaïne, midazolam, et leurs sels, leurs esters, leurs hydrates, leurs polymorphes et leurs isomères.

Des antiasthmatiques sont par exemple : ablukast, azélastine, bunaprolast, cinalukast, cromitrile, cromolyne, énofélast, isambxole, kétotifène, levcromékaline, lodoxamide, montélukast, ontazolast, oxarbazole, oxatomide, piriprost potassium, pirolate, pobilukast, édamine, pranlukast, quazolast, répirinast, ritolukast, sulukast, tétrazolastméglumine, tiaramide, tibénélast, tomélukast, tranilast, verlukast, vérofylline, zarirlukast, et leurs sels, leurs esters, leurs hydrates, leurs polymorphes et leurs isomères.

Des agents anticancéreux sont par exemple : adriamycine, aldesleukine, allopurinol, altrétamine, amifostine, anastrozole, asparaginase, bétaméthasone, bexarotène, bicalutamide, bléomycine, busulfan, capécitabine, carboplatine, carmustine, chlorambucil, cisplatine, cladribine, oestrogène conjugué, cortisone, cyclophosphamide, cytarabine, dacarbazine, daunorubicine, dactinomycine, dénileukine, dexaméthasone, discodermolide, docétaxel, doxorubicine, éloposidem, épirubicine, époétine, épothilones, estramustine, oestrogène estérifié, éthinyl-oestradiol, étoposide, exemestane, flavopirdol, fluconazole, fludarabine, fluorouracile, flutamide, floxuridine, gemcitabine, hexaméthylmélamine, hydrocortisone, hydroxyurée, ifosfamide, lémiposide, létrozole, leuprolide, lévamisole, lévothyroxine, lomustine, méchloréthamine, melphalan, mercaptopurine, mégestrol, méthotrexate, méthylprednisolone, méthyltestostérone, mithramycine, mitomycine, mitotane, mitoxantrone, mitozolomide, mutamycine, nilutamide, pamidronate, pentostatine, plicamycine, porfimer, prednisolone, procarbazine, sémustine, streptozocine, tamoxifen, témozolamide, téniposide, testolactone, thioguanine, tomudex, torémifène, trétinoïne, sémustine, streptozolocine, verteprofine, vinblastine, vincristine, vindesine, vinorelbine, et leurs sels, leurs esters, leurs hydrates, leurs polymorphes et leurs isomères.

Des anticoagulants et des antithrombotiques sont par exemple : warfarine, danaparoïde, alprostadil, anagralide, argatroban, ataprost, bétaprost, camonagrel, cilostazol, clinprost, clopidogrel, cloricromène, dermatan, désirudine, domitroban, drotavérine, époprosténol, fradafiban, gabexate, iloprost, isbogrel, lamifiban, léfradafiban, lépirudin, lévosimendan, lexipafant, mélagatran, nafagrel, nafamostat, nizofenone, orbifiban, ozagrel, pamicogrel, quinobendan, sarpogralate, satigrel, simendan, ticlopidine, vapiprost, tirofiban, xemilofiban, Y20811, et leurs sels, leurs esters, leurs hydrates, leurs polymorphes et leurs isomères.

Des anticonvulsivants sont par exemple : carbamazépine, clonazepam, clorazépine, diazépam, divalproex, éthosuximide, éthotion, felbamate, fosphénytoïne, gabapentine, lamotrigine, lévétiracétam, lorazépam, méphénytoïne, méphobarbital, métharbital, méthsuximide, oxcarbazépine, phénobarbital, phénytoïne, prégabaline, primidone, tiagabine, topiramate, acide valproïque, vigabatrine, zonisamide, et leurs sels, leurs esters, leurs hydrates, leurs polymorphes et leurs isomères.

Des antidiabétiques sont par exemple : acarbose, acétohexamide, carbutamide, chlorpropamide, épalrestat, glibornuride, gliclazide, glimépiride, glipizide, gliquidone, glisoxepide, glyburide, glyhexamide, metformine, miglitol, natéglinide, orlistat, phénbutamide, pioglitazone, répaglinide, rosiglitazone, tolazamide, tolbutamide, tolcyclamide, tolrestat, troglitazone, voglibose, et leurs sels, leurs esters, leurs hydrates, leurs polymorphes et leurs isomères.

Des antiémétiques sont par exemple : alprazolam, benzquinamide, benztropine, bétahistine, chlorpromazine, dexaméthasone, difénidol, dimenhydrinate, diphénhydramine, dolasétron, dompéridone, dronabinol, dropéridol, granisétron, halopéridol, lorazépam, meclizine, méthylprednisolone, métoclopramide, ondansétron, perphénazine, prochlorpérazine, promethazine, scopolamine, tributine, triéthylpérazine, triflupromazine, triméthobenzamide, tropisétron, et leurs sels, leurs esters, leurs hydrates, leurs polymorphes et leurs isomères.

Des agents antiglaucome sont par exemple : alprénoxime, dapiprazole, dipivéfrine, latanoprost, naboctate, pimabine, et leurs sels, leurs esters, leurs hydrates, leurs polymorphes et leurs isomères.

Des antihistaminiques ou des bêta agonistes sont par exemple : acépromazine, acrivastine, activastine, albutérol, alimémazine, antazoline, azélastine, bitoltérol, amlexanox, benzydamine, bromphéniramine, cétirizine, chlorphéniramine, cimétidine, cinnarizine, clémastine, clofédanol, cycloheptazine, cyproheptadine, difencloxazine, diphénhydramine, dotarizine, éphédrine, épinastine, épinéphrine, éthylnorépinéphrine, fenpentadiol, fenpotérol, fexofénadine, flurbiprofène, hydroxyzine, isoétharine, isoprotérénol, kétorolac, lévocétirizine, lévomépromazine, loratidine, méquitazine, métaprotérénol, niaprazine, oxatomide, oxomémazine, phényléphrine, phénylpropanolamine, pirbutérol, prométhazine, pseudoéphédrine, pyrilamine, ranitidine, salmétérol, terbutaline, terfénadine, tranilast, dérivés de la xanthine ; et leurs sels, leurs esters, leurs hydrates, leurs polymorphes et leurs isomères.

Des agents anti-infectieux, notamment les antibiotiques, les antifongiques, les antiviraux, sont par exemple : abacavir, aciclovir, albendazole, amantadine, amphotéricine, amikacine, acide aminosalicylique, amoxicilline, ampicilline, amprénavir, atovaquine, azithromycine, aztréonam, céfaclor, céfadroxil, céfazoline, cefdinir, céfixime, cefpodoxime proxetil, cefprozil, ceftibutène, céphalexine, chloroquine, cidofovir, cilastatine, ciprofloxacine, clarithromycine, acide clavulanique, clindamycine, dalfopristine, dapsone, delavirdine, déméclocycline, didanosine, doxycycline, éfavirenz, énoxacine, érythromycine, éthambutol, éthionamide, famciclovir, fluconazole, flucytocine, foscarnet, ganciclovir, gatifloxacine, griséofulvine, hydroxychloroquine, indinavir, isoniazide, itraconazole, ivermectil, kétoconazole, lamivudine, lévofloxacine, linizolide, loméfloxacine, loracarbef, mébendazole, méfloquine, méthanamine, métronidazole, minocycline, moxifloxacine, acide nalidixique, nelfinavir, néomycine, névirapine, nitrofurantoïne, norfloxacine, ofloxacine, oseltamivir, oxytétracycline, pénicilline V, péfloxacine, praziquantel, pyrazinamide, pyriméthamine, quinidine, quinupristine, rétonavir, ribavirine, rifabutine, rifampicine, rimantadine, saquinavir, sparfloxacine, stavudine, streptomycine, sulfaméthoxazole, tétramycine, terbinafine, tétracycline, thiabendazole, tobramycine, triméthoprime, troléandomycine, trovafloxacine, valaciclovir, vancomycine, zalcitabine, zanamivir, zidovudine, et leurs sels, leurs esters, leurs hydrates, leurs polymorphes et leurs isomères.

Des antiparkinsoniens sont par exemple : amantadine, adrogolide, altinicline, benzatropine, bipéridène, brasofensine, bromocriptine, budipine, cabergoline, CHF-1301, dihydrexidine, entacapone, etilevodopa, idazoxane, iométopane, lazabémide, melevodopa, carbidopa, levodopa, mofégiline, moxiraprine, pergolide, pramipexole, quinélorane, rasagiline, ropinirole, séligiline, talipexole, tolcapone, trihexyphenidyle, et leurs sels, leurs esters, leurs hydrates, leurs polymorphes et leurs isomères.

Des agents antirhumatismaux sont par exemple : azathioprine, bétaméthasone, célécoxib, cyclosporine, diclofénac, hydroxychloroquine, indométhacine, acide mercaptobutanedioïque, méthylprednisolone, naproxène, pénicillamine, piroxicam, prednisolone, sulfasalazine, et leurs sels, leurs esters, leurs hydrates, leurs polymorphes et leurs isomères.

Des antiagrégants plaquettaires sont par exemple : anagrélide, aspirine, cilostazol, clopidogrel, dipyridamole, époprosténol, éptifibatide, ticlopidine, tinofiban, et leurs sels, leurs esters, leurs hydrates, leurs polymorphes et leurs isomères.

Des antispasmodiques et des anticholinergiques sont par exemple : aspirine, atropine, diclofénac, hyoscyamine, mésoprostol, méthocarbamol, phénobarbital, scopolamine, et leurs sels, leurs esters, leurs hydrates, leurs polymorphes et leurs isomères.

Des antitussifs sont par exemple : paracétamol, acrivastine, benzonatate, béractant, bromphéniramine, caféine, calfactant, carbétapentane, chlorphéniramine, codéine, colfuscérine, dextromethorphan, doxylamine, fexofénadine, guaphénésine, métaprotérénol, montélukast, pentoxyphilline, phényléphrine, phénylpropano lamine, pirbutérol, pseudoéphédrine, pyrilamine, terbutaline, théophylline, zafirlukast, zileuton et leur sels, leurs esters, leurs hydrates, leurs polymorphes et leurs isomères.

Des inhibiteurs de l'anhydrase carbonique sont par exemple : acétazolamide, dichlorphénamide, dorzolamide, méthazolamide, sézolamide, et leurs sels, leurs esters, leurs hydrates, leurs polymorphes et leurs isomères.

Des agents cardiovasculaires, notamment les hypolipémiants, les anti-arythmiques, les vasodilatateurs, les antiangineux, les anti-hypertenseurs, les vasoprotecteurs, sont par exemple : acébutolol, adénosine, amiodarone, amiloride, amlodipine, nitrate d'amyle, aténolol, atorvastatine, benzépril, bépiridil, bétaxalol, bisoprolol, candésartan, captopril, carténolol, carvédilol, cérivastatine, chlorthalidone, chlorthiazole, clofibrate, clonidine, colestipol, colosévélam, digoxine, diltiazem, disopyramide, dobutamine, dofétilide, doxazosine, énalapril, époprosténol, éprosartan, esmolol, éthacrynate, érythrityl, félodipine, fénoidapam, fosinopril, flécaïnide, furosémide, fluvastatine, gemfibrozil, hydrochlorthiazide, hydroflumethazine, ibutilide, indapamide, isosorbide, irbésartan, labétolol, lacidipine, lisinopril, losartan, lovastatine, mécamylamine, métoprolol, métarminol, métazolone, méthylchlothiazide, méthyldopa, métyrosine, mexilétine, midrodine, milrinone, moexipril, nadolol, niacine, nicardipine, nicorandil, nifédipine, nimodipine, nisoldipine, nitroglycérine, phénoxybenzamine, périndopril, polythiazide, pravastatine, prazosine, procaïnamide, propafénone, propranolol, quanfacine, quinapril, quinidine, ranipril, simvastatine, sotalol, spironolactone, telmisartan, térazosine, timolol, tocaïnamide, torsémide, trandolapril, triamtérène, trapidil, valsartan, et leurs sels, leurs esters, leurs hydrates, leurs polymorphes et leurs isomères.

Des vasodilatateurs sont par exemple : adénosine, alvérine, caféine, dihydroergocornine, énalapril, énoximone, iloprost, kalléone, lidoflazine, nicardipine, nimodipine, acide nicotinique, papavérine, pilocarpine, salbutamol, théophylline, trandolapril, uradipil, vincamine, et leurs sels, leurs esters, leurs hydrates, leurs polymorphes et leurs isomères.

Des inhibiteurs de cholinestérase sont par exemple : donépézil, néostigmine, pyridostigmine, rivastigmine, tacrine, et leurs sels, leurs esters, leurs hydrates, leurs polymorphes et leurs isomères.

Des stimulants du système nerveux central sont par exemple : caféine, doxapram, dexoamphétamine, donépézil, méthamphétamine, méthylphénidate, modafinil, néostigmine, pémoline, phentermine, pyridostigmine, rivastigmine, tacrine et leur sels, leurs esters, leurs hydrates, leurs polymorphes et leurs isomères.

Des contraceptifs sont par exemple : désogestral, éthinyl-oestradiol, éthynodiol, lévonorgestrel, médroxyprogestérone, mestranol, norgestimate, noréthindrone, norgestrel, et leurs sels, leurs esters, leurs hydrates, leurs polymorphes et leurs isomères.

Des agents de traitement de la mucoviscidose sont par exemple : pancrélipase, tobramycine, et leurs sels, leurs esters, leurs hydrates, leurs polymorphes et leurs isomères.

Des agonistes des récepteurs de la dopamine sont par exemple : amantadine, cabergoline, fenoldopam, pergolide, pramipezal, ropinirole et leurs sels, leurs esters, leurs hydrates, leurs polymorphes et leurs isomères.

Des agents de traitement de l'endométriose sont par exemple : danazol, norethindrone, et leurs sels, leurs esters, leurs hydrates, leurs polymorphes et leurs isomères.

Des agents de traitement des dysfonctionnements érectiles sont par exemple, sildénafil, tadalafil, vardénafil, yohimbine, et leurs sels, leurs esters, leurs hydrates, leurs polymorphes et leurs isomères.

Des agents de traitement de la fertilité sont par exemple : clomiphène, progésterone, et leurs sels, leurs esters, leurs hydrates, leurs polymorphes et leurs isomères.

Des agents de traitement de troubles gastro-intestinaux sont par exemple : alosétron, bisacodyl, subsalicylate de bismuth, célécoxib, cimétidine, difoxine, diphénoxylate, docusate, ésoméprazole, famotidine, glycopyrrolate, lansoprazole, lopéramide, métoclopramide, nizatidine, oméprazole, pantoprazole, rabéprazole, ranitidine, siméthicone, sucralfate, et leurs sels, leurs esters, leurs hydrates, leurs polymorphes et leurs isomères.

Des immunomodulateurs et des immunosuppresseurs sont par exemple : azathioprine, ceftizoxime, cyclosporine, leflunomide, lévamisol, mycophénolate, phthalidomide, ribavirine, sirolimus, tacrolimus et leurs sels, leurs esters, leurs hydrates, leurs polymorphes et leurs isomères.

Des agents de traitements de la maladie d'Alzheimer sont par exemple : CP 118954, donépézil, galanthamine, métrifonate, révastigmine, tacrine, TAK-147, et leurs sels, leurs esters, leurs hydrates, leurs polymorphes et leurs isomères.

Des antimigraineux sont par exemple : paracétamol, dihydroergotamine, divalproex, ergotamine, propranolol, risatriptan, sumatriptan, trimetrexate, et leurs sels, leurs esters, leurs hydrates, leurs polymorphes et leurs isomères.

Des relaxants des muscles sont par exemple : azapropazone, baclofène, carisoprodol, dérivés de quinine, chloromézanone, chlorophénésincarbamate, chlorozoxazone, cyclobenzaprine, dantrolène, chlorure de dimethyltubocurarinium, fényramidol, guaiphensine, mémantin, méphénésine, méprobamate, métamisol, métaxalone, méthocarbamol, orphénadrine, phénazone, phénprobamate, tétrazépam, tizanidine, tybamate et leurs sels, leurs esters, leurs hydrates, leurs polymorphes et leurs isomères.

Des analogues de nucléoside sont par exemple : abacavir, aciclovir, didanosine, gamciclovir, gemcitabine, lamivudine, ribavirine, stavudine, zalcitabine, et leurs sels, leurs esters, leurs hydrates, leurs polymorphes et leurs isomères.

Des agents de traitement de l'ostéoporose sont par exemple : alendronate, calcitonine, oestradiol, estropipate, médroxyprogestérone, noréthindrone, norgestimate, pamidronate, raloxifène, risdronate, zolédronate, et leurs sels, leurs esters, leurs hydrates, leurs polymorphes et leurs isomères.

Des parasympathomimétiques sont par exemple : béthanechol, bipéridine, édrophonium, glycopyrolate, hyoscyamine, pilocarpine, tacrine, yohimbine, et leurs sels, leurs esters, leurs hydrates, leurs polymorphes et leurs isomères.

Des prostaglandines sont par exemple : alprostadil, époprosténol, misoprostol, et leurs sels, leurs esters, leurs hydrates, leurs polymorphes et leurs isomères.

Des agents psychothérapeutiques sont par exemple : acétophénazine, alentémol, alpertine, alprazolam, amitriptyline, apriprazole, azapérone, batélapine, béfipiride, benpéridol, benzindopyrine, bimithil, biripérone, brofoxine, brompéridol, bronipéridol, bupropion, buspirone, butaclamol, butapérazine, carphénazine, carvotroline, chlorazépine, chlordiazépoxide, chlorpromazine, chlorprothixène, cinpérène, cintriamide, citalopram, clomacrane, clonazépam, clopenthixol, clopimozide, clopipazane, cloropérone, clothiapine, clothixamide, clozapine, cyclophénazine, dapiprazole, dapoxétine, désipramine, divalproex, dipyridamole, doxépine, dropéridol, duloxétine, eltoprazine, eptipirone, étazolate, fénimide, flibansérine, flucindole, flumézapine, fluoxétine, fluphénazine, fluspipérone, fluspirilène, flutroline, fluvoxamine, gépirone, gévotroline, halopémide, halopéridol, hydroxyzine, hydroxynortriptyline, ilopéridone, imidoline, lamotrigine, loxapine, enpérone, mazapertine, méphobarbital, méprobamate, mésoridazine, mésoridazine, milnacipran, mirtazépine, métiapine, milenpérone, milipertine, molindone, nafadotride, naranol, nefazodone, neflumozide, ocapéridone, odapipam, olanzapine, oxethiazine, oxipéromide, pagoclone, palipéridone, paroxitène, penfluridol, pentiapine, perphénazine, phénelzine, pimozide, pinoxépine, pipampérone, pipéracétazine, pipotiazine, piquindone, piracétam, pirlindole, pivagabine, pramipexole, prochlorpérazine, promazine, quétiapine, réboxetine, rémoxipride, rispéridone, rimcazole, robolzotan, sélégiline, sépéridol, sertraline, sertindole, seteptiline, sétopérone, spipérone, sunipitrone, tépirindole, thioridazine, thiothixène, tiapride, tiopéridone, tiospirone, topiramate, tranylcypromine, trifluopérazine, triflupéridol, triflupromazine, trimipramine, venlafaxine, ziprasidone, et leurs sels, leurs esters, leurs hydrates, leurs polymorphes et leurs isomères.

Des sédatifs, des hypnotiques et des tranquillisants sont par exemple : bromazépam, buspirone, clazolam, clobazam, chlorazépate, diazépam, démoxépam, dexmédétomidine, diphényhydramine, doxylamine, enciprazine, estrazolam, hydroxyzine, ketazolam, lorazatone, lorazépam, loxapine, médazépam, mépéridine, méthobarbital, midazolam, nabilone, nisobamate, oxazépam, pentobarbital, prométhazine, propofol, triazolam, zaléplon, zolpidem, et leurs sels, leurs esters, leurs hydrates, leurs polymorphes et leurs isomères.

Des agents de traitement dermatologiques sont par exemple : acitrétine, alclométasone, alitretinoïne, bétaméthasone, calciprotrine, clobétasol, clocortolone, clotrimazole, cyclosporine, désonide, difluorosone, doxépine, eflomithine, finastéride, flurandrénolide, hydrochloroquine, hydroquinone, hydroxyzine, kétoconazole, mafénide, malathion, ménobenzone, néostigmine, nystatine, podophyllotoxine, povidone, tazorotène, trétinoïne, et leurs sels, leurs esters, leurs hydrates, leurs polymorphes et leurs isomères.

Des stéroïdes et des hormones sont par exemple : alclométasone, bétaméthasone, citrorélix, clobétasol, clocortolone, les cortisones, danazol, désonide, désogestrel, désoximétasone, dexaméthasone, diflorasone, oestradiol, oestrogènes, estropipate, éthynilestradiol, fluocinolone, flurandrénolide, fluticasone, halobétasol, hydrocortisone, leuprolide, lévonorgestrel, lévothyroxine, médroxyprogestérone, méthylprednisolone, méthyltestosterone, mométasone, noréthindrone, norgestrel, oxandrolone, oxymétholone, prednicarbate, prednisolone, progestérone, stanozolol, testostérone, et leurs sels, leurs esters, leurs hydrates, leurs polymorphes et leurs isomères.

On peut également se référer à la liste de PA donnés dans la demande EP 0 609 961 aux pages 4 à 8. Le PA mis en oeuvre appartient par exemple à au moins l'une des familles de substances actives suivantes : amphétamines, analgésiques, anorexigènes, antalgiques, antidépresseurs, antiépileptiques, antimigraineux, antiparkinsoniens, antitussifs, anxiolytiques, barbituriques, benzodiazépines, hypnotiques, laxatifs, neuroleptiques, opiacés, psychostimulants, psychotropes, sédatifs, stimulants. Dans le cas où le PA est un PA analgésique (PAa), il s'agit, de préférence, d'un opioïde.

Plus précisément encore, le PA mis en oeuvre est choisi parmi les composés suivants : anileridine, acetorphine, acetylalphamethylfentanyl, acétyldihydrocodéine, acetylmethadol, alfentanil, allylprodine, alphacetylmethadol, alphameprodine, alphaprodine, alphamethadol, alphamethylfentanyl, alpha-methylthio-fentanyl, alphaprodine, anileridine, butorphanol, benzethidine, benzylmorphine, beta-hydroxyfentanyl, beta-hydroxy-methyl-3-fentanyl, betacetylmethadol, betameprodine, betamethadol, betaprodine, bezitramide, buprenorphine, butyrate de dioxaphetyl, clonitazene, cyclazocine, cannabis, cetobemidone, clonitazene, codéine, coca, cocaïne, codoxime, dezocine, dimenoxadol, dioxaphetylbutyrate, dipipanone, desomorphine, dextromoramide, dextropropoxyphène, diampromide, diéthyl-thiambutene, difenoxine, dihydrocodéine, dihydroetorphine, dihydromorphine, dimenoxadol, dimepheptanol, dimethylthiambutene, diphenoxylate, dipipanone, dronabinol, drotebanol, eptazocine, ethoheptazine, ethylmethylthiambutene, ethylmorphine, etonitazene, ecgonine, ephedrine, ethylmethylthiambutene, ethylmorphine, etonitazene, etorphine, etoxeridine, fentanyl, furethidine, héroïne, hydrocodone, hydromorphinol, hydromorphone, hydroxypethidine, isomethadone, ketobemidone, levallorphane, lofentanil, levomethorphane, levomoramide, levophenacylmorphane, levorphanol, meptazinol, meperidine, metazocine, methadone, methyldesorphine, methyldihydro-morphine, methylphenidate, methyl-3-thiofentanyl, methyl-3-fentanyl, metopon, moramide, morpheridine, morphine, myrophine, nabilone, nalbuphine, narceine, nicomorphine, norlevorphanol, normethadone, nalorphine, normorphine, nicocodine, nicodicodine, nicomorphine, noracymethadol, norcodeine, norlevorphanol, normethadone, normorphine, norpipanone, opium, oxycodone, oxymorphone, phenadoxone, phenoperidine, promedol, properidine, propiram, propoxyphene, parafluorofentanyl, pentazocine, pethidine, phenampromide, phenazocine, phenomorphane, phenoperidine, pholcodine, piminodine, piritramide, proheptazine, propranolol, properidine, propiram, racemethorphane, racemoramide, racemorphane, remifentanil, sufentanil, tétrahydrocannabinol, thebacone, thébaïne, thiofentanyl, tilidine, trimeperidine, tramadol, et leurs sels, leurs esters, leurs hydrates, leurs polymorphes et leurs isomères pharmaceutiquement acceptables, et leurs mélanges.

Parmi les PA anti-inflammatoires envisageables, on peut citer : célécoxib, ibuprofène, paracétamol, diclofénac, naproxène, benoxaprofène, flurbiprofène, fénoprofène, flubufène, ketoprofène, indoprofène, piroprofène, carprofène, oxaprozine, pramoprofène, muroprofène, trioxaprofène, suprofène, amineoprofène, acide tiaprofenique, fluprofène, acide bucloxique, indométhacine, sulindac, tolmetine, zomepirac, tiopinac, zidometacine, acémétacine, fentiazac, clidanac, oxpinac, acide méfénamique, acide méclofénamique, acide flufénamique, acide niflumique, acide tolfénamique, diflurisal, flufénisal, piroxicam, sudoxicam ou isoxicam, et leurs sels, leurs esters, leurs hydrates, leurs polymorphes et leurs isomères pharmaceutiquement acceptables, et leurs mélanges.

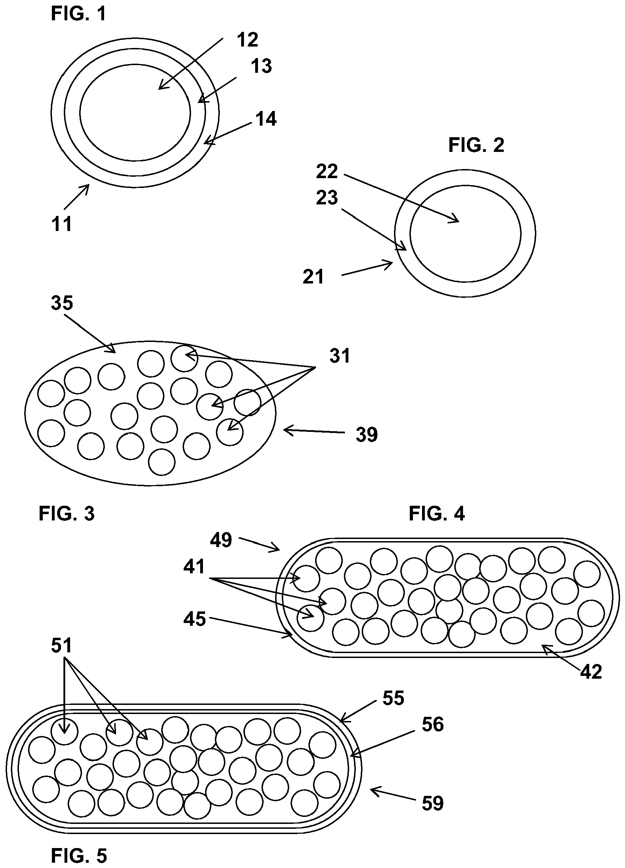

Les exemples ci-après sont donnés uniquement à titre d'illustration et peuvent permettre de comprendre les formes pharmaceutiques décrites et de faire ressortir ses variantes de réalisation et/ou de mise en oeuvre, ainsi que ses différents avantages. Divers modes de réalisation sont illustrés à titre d'exemples non limitatifs aux figures 1 à 5.
La figure 1 représente une microparticule 11 dont le coeur de PA 12 est enrobé d'un enrobage 13 sur lequel est déposé l'agent D 14. L'enrobage 13 contient le polymère A, l'agent plastifiant B et éventuellement l'agent tensioactif C.
La figure 2 représente une microparticule 21 dont le coeur de PA 22 contient un agent D1. Le coeur de PA 22 est enrobé d'un enrobage 23 qui contient également un agent D2. Les agents D1 ou D2 peuvent être identiques ou différents l'un de l'autre.
La figure 3 représente un pellet ou granule 39, par exemple obtenu par extrusion, qui contient des microparticules 31 dans une phase liante 35 contenant au moins un agent D. Les microparticules 31 comprennent des microparticules réservoirs et éventuellement des microparticules non enrobées de PA.
La figure 4 représente un comprimé 49 contenant des microparticules 41, par exemple des microparticules réservoirs et éventuellement des microparticules à libération immédiate, dans un liant 42 contenant un agent D2. Le comprimé 49 est revêtu d'un revêtement 45 contenant un agent D1. Les agents D1 ou D2 peuvent être identiques ou différents l'un de l'autre.
La figure 5 représente une gélule 59 dont la paroi 56 est enrobée d'un revêtement 55 à base d'un agent D. La gélule 59 contient des microparticules 51, par exemple des microparticules réservoir et éventuellement des microparticules à libération immédiate.

### EXEMPLES

### Exemple 1 : Gélules d'aciclovir - l'agent D est contenu dans le support neutre des

### microparticules

### Etape 1 :

288 g d'aciclovir et 72 g d'hydroxypropyl cellulose (Klucel EF® / Aqualon) sont dispersés dans 840 g d'eau. La suspension est pulvérisée sur 240 g de gomme guar (Danisco) en lit d'air fluidisé (Glatt GPCG1).

### Etape 2 :

1,4 g d'éthylcellulose (Ethocel 20 Premium / Dow), 9,24 g de cellulose acétate butyrate (CAB 171-15 /Eastman), 1,68 g de polysorbate 80 (Tween 80 / Uniqema) et 1,68 g de triéthylcitrate (Morflex) sont solubilisés dans un mélange composé de 94% d'acétone et 6% d'eau. Cette solution est pulvérisée sur 56 g de granulé d'aciclovir (préparé à l'étape 1).

Les microparticules obtenues sont ensuite placées dans une gélule en gélatine de taille 0 (de manière à avoir une dose d'aciclovir de 150 mg par gélule).

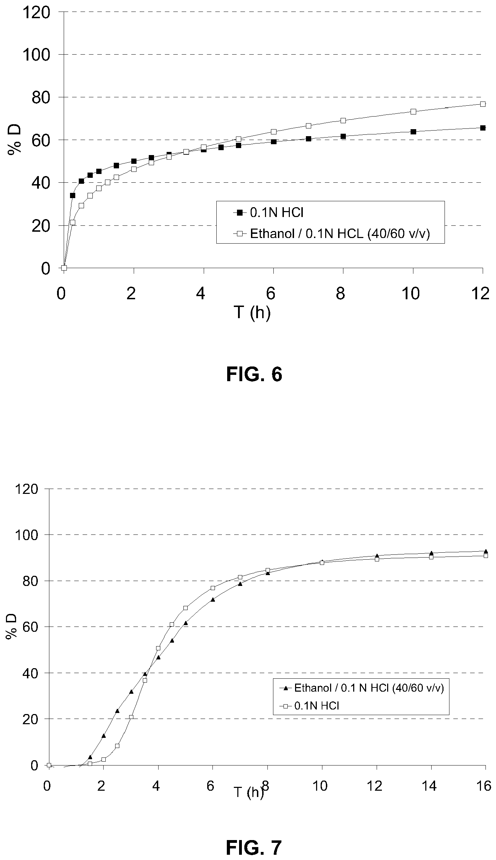

Les profils de dissolution D (%) en fonction du temps (h) dans 900 ml d'HCl 0,1 N et dans 500 ml d'un mélange éthanol/HCl 0,1 N (40/60 v/v) sous une agitation à palettes à 75 tours/min sont donnés en Figure 6 :
On constate que les profils de dissolution dans les milieux HCl 0,1 N et éthanol / HCl 0,1 N (40/60 v/v) sont très similaires. En particulier, il n'y a pas d'accélération sensible de la quantité libérée en présence d'éthanol (donc pas de « dose dumping »).

### Exemple 2 : Gélule de metformine - l'agent D est contenu dans le revêtement de la gélule Etape 1 :

500 g de metformine sont dispersés dans 2586 g d'eau. La solution est pulvérisée sur 450 g de sphères de cellulose (Asahi-Kasei) dans un Glatt GPCG1.

### Etape 2 :

228 g d'éthylcellulose (Ethocel 20 Premium / Dow), 30 g de povidone (Plasdone K29-32 / International Specialty Products Inc.), 12 g d'huile de ricin hydrogénée polyoxyl-40 (polyoxyéthylèneglycéroltrihydroxystéarate : Cremophor RH 40 / ISP) et 30 g d'huile de ricin sont solubilisés dans un mélange composé de 60% d'acétone et 40% d'isopropanol. Cette solution est pulvérisée sur 700 g de granulés de metformine préparés à l'étape 1.

Les microparticules obtenues sont ensuite placées dans une gélule en gélatine de taille 2 (de manière à avoir une dose de metformine de 150 mg par gélule). Cette gélule est ensuite pelliculée avec une solution de carboxyméthylcellulose de sodium (Blanose 7 LF / Aqualon) à hauteur de 20 mg de carboxyméthylcellulose de sodium pour 60 mg de gélatine.

Les profils de dissolution dans 900 ml de HCl 0,1 N et dans 500 ml d'un mélange éthanol / HCl 0,1 N (40/60 v/v) sous une agitation à palettes à 75 tours/min sont donnés en Figure 7 :

On constate que les profils de dissolution dans les milieux HCl 0,1 N et éthanol / HCl 0,1 N (40/60 v/v) sont très similaires. En particulier, il n'y a pas d'accélération sensible de la quantité libérée en présence d'éthanol (donc pas de « dose dumping »).

### Exemple 3 : Gélules d'aciclovir - l'agent D est contenu dans le support neutre des microparticules et dans le constituant de la gélule.

### Etape 1 :

288 g d'aciclovir et 72 g d'hydroxypropylcellulose (Klucel EF® / Aqualon) sont dispersés dans 840 g d'eau. La suspension est pulvérisée sur 240 g de gomme guar (Danisco) dans un Glatt GPCG1.

### Etape 2 :

9,84 g d'éthylcellulose (Ethocel 20 Premium / Dow), 0,24 g de povidone (Plasdone K29/32 / ISP), 0,24 g de sorbitan mono-oléate (Span 80 / Uniqema) et 1,68 g d'huile de ricin (Garbit Huilerie) sont solubilisés dans un mélange composé de 60% d'acétone et 40% d'isopropanol. Cette solution est pulvérisée sur 48 g de granulé d'aciclovir (préparé à l'étape 1).

Les microparticules obtenues sont ensuite placées dans une gélule végétale (à base d'hypromellose [ou HPMC]) de taille 0 (de manière à avoir une dose d'aciclovir de 150 mg par gélule).

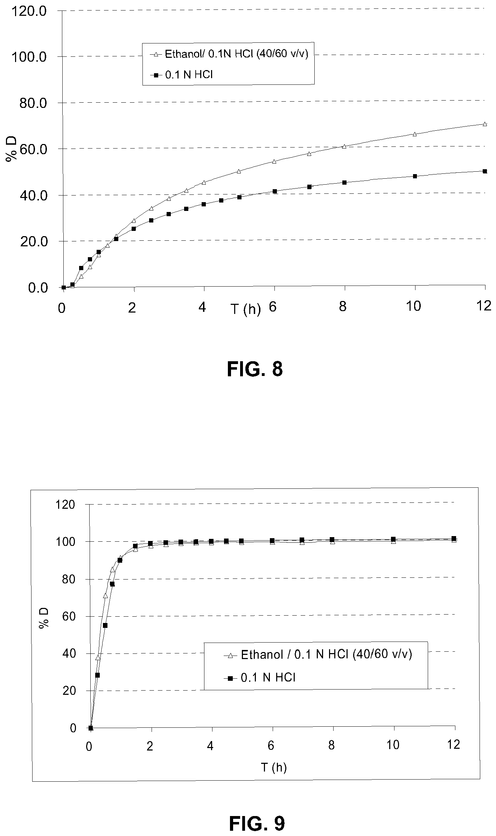

Les profils de dissolution dans 900 ml de HCl 0,1 N et dans 500 ml d'un mélange éthanol/HCl 0,1 N (40/60 v/v) sous une agitation à palettes à 75 tours/min sont donnés en Figure 8 :
On constate que les profils de dissolution dans les milieux HCl 0,1 N et éthanol/HCl 0,1 N (40/60 v/v) sont très similaires. En particulier, il n'y a pas d'accélération sensible de la quantité libérée en présence d'éthanol (donc pas de « dose dumping »).

### Exemple 4 : Gélule de metformine - L'agent D est mélangé avec les microparticules

### Etape 1 :

350 g de metformine, 50 g d'hydroxypropylcellulose (Klucel EF® / Aqualon) et100 g de glycolate sodique d'amidon (Primojel / Avebe) sont dispersés dans 700 g d'eau et 467 g d'éthanol. La solution est pulvérisée sur 500 g de gomme guar (Danisco) dans un Glatt GPCG1.

### Etape 2 :

224 g d'éthylcellulose (Ethocel 20 Premium / Dow), 5,2 g de sorbitan mono-oléate (Span 80 / Uniqema) et 31,2 g d'huile de ricin (Garbit Huilerie) sont solubilisés dans un mélange composé de 60% d'acétone et 40% d'isopropanol. Cette solution est pulvérisée sur 390 g de granulé de metformine (préparé à l'étape 1).

### Etape 3 :

200 g de microparticules obtenues à l'issue de l'étape 2 sont mélangés avec 65 g de mannitol (Pearlitol SD 200), 30 g d'hypromellose [ou HPMC] (Methocel E5), 5 g de stéarate de magnésium et env. 60 g d'eau et extrudées sur une grille de 1,5 mm (extrudeuse Fitzpatrick MG-55). Les bâtonnets obtenus sont ensuite sphéronisés sur un plateau ayant une rugosité de 1 mm à une vitesse de 1500 tours/min (sphéroniseur de laboratoire Fitzpatrick Q-230.T).

Les microparticules obtenues sont ensuite placées dans une gélule en gélatine de taille 0 (de manière à avoir une dose de metformine de 80 mg par gélule).

Les profils de dissolution dans 900 ml de HCl 0,1 N et dans 500 ml d'un mélange éthanol/HCl 0,1 N (40/60 v/v) sous une agitation à palettes à 75 tours/min sont donnés en Figure 9.

On constate que les profils de dissolution dans les milieux HCl 0,1 N et éthanol/HCl 0,1 N (40/60 v/v) sont très similaires.

75 % env. de PA est libéré dans les deux cas en 45 min, ce qui représente la frontière des formes MR. Pour ralentir encore la libération du PA, l'homme du métier pourra notamment augmenter la taille des microparticules, ou augmenter le taux d'enrobage.

### Exemple 5 : Comportement des agents D dans les solutions aqueuses et alcooliques.

Différents composés D sont introduits dans un flacon contenant soit de l'eau (à gauche sur les Figures), soit une solution éthanol/eau dans le rapport 40/60 v/v (flacon de droite sur les Figures).

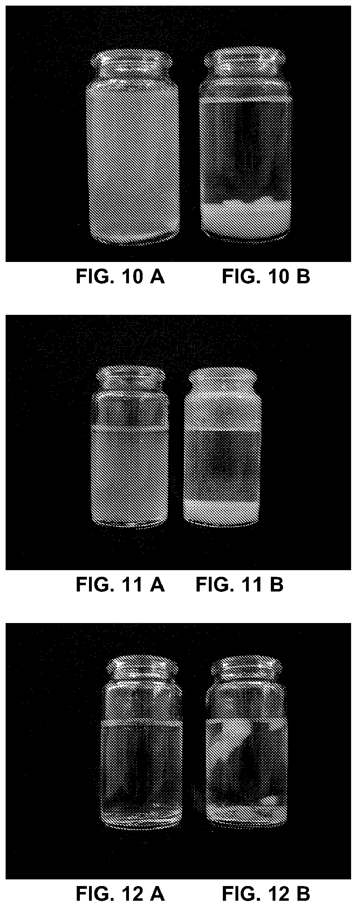

La Figure 10 montre l'aspect obtenu en 15 min dans le cas d'une substance insoluble dans l'eau et dans l'éthanol - ici l'amidon glycolate de sodium (Primojel® / Avebe), mais qui gonfle plus dans l'eau qu'en solution alcoolique.

La Figure 11 montre le cas d'une substance soluble dans l'eau mais pas dans le mélange eau/éthanol, ici de la gomme guar (Grindsted® Guar / Danisco)

La Figure 12 montre l'aspect obtenu en 30 min dans le cas d'une substance dont la vitesse de solubilité est plus élevée dans l'eau que dans le mélange eau/éthanol, ici de l'hypromellose [ou HPMC] (Methocel® E5 / Dow).

### Exemple 6 : Gélule de metformine - L'agent D est mélangé avec les microparticules

### Etape 1 :

1700 g de metformine sont solubilisés dans 2348 g d'eau. La solution est pulvérisée sur 300 g de sphères de cellulose (Cellets 90 / Pharmatrans) dans un Glatt GPCG1.

### Etape 2 :

249,6 g d'éthylcellulose (Ethocel 20 Premium / Dow), 19,2 g de povidone (Plasdone K29/32 / ISP), 12,8 g de polyoxyl-40 huile de ricin hydrogénée (Cremophor RH 40 / BASF) et 38,4 g d'huile de ricin (Garbit Huilerie) sont solubilisés dans un mélange composé de 60 % d'acétone et 40 % d'isopropanol. Cette solution est pulvérisée sur 480 g de granulé de metformine (préparé à l'étape 1).

### Etape 3 :

6 g de microparticules obtenues à l'issue de l'étape 2 sont mélangés avec 0,4 g d'hypromellose [ou HPMC] (Methocel E4M / Colorcon), 0,2 g d'hydroxypropylcellulose (Klucel HXF / Aqualon) et 0,04 g de stéarate de magnésium à l'aide d'un mélangeur type roue Rhön (Mini 80 / J. Engelsmann AG) pendant 30 min. Le mélange obtenu est ensuite placé dans une gélule en gélatine de taille 0 (de manière à avoir une dose de metformine d'environ 150 mg par gélule).

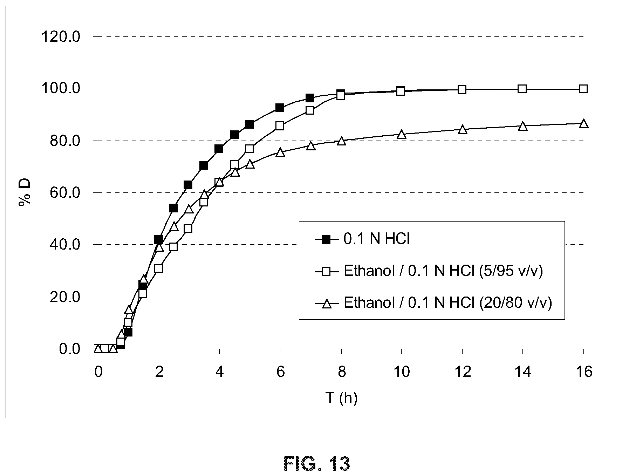

Les profils de dissolution dans 900 ml de HCl 0,1 N, 900 ml de mélange éthanol / HCl 0,1 N (5/95 v/v) et 900 ml de mélange éthanol / HCl 0,1 N (20/80 v/v) sous une agitation à palettes à 75 tours/min sont donnés en Figure 13.

On constate que le profil de dissolution dans le milieu HCl 0,1 N est similaire ou plus rapide que dans les milieux contenant de l'éthanol.

## Revendications

1. Forme pharmaceutique ou diététique orale comprenant des microparticules de type réservoir à libération prolongée d'au moins un principe actif (PA) et au moins un agent D qui est un composé pharmaceutiquement acceptable dont la vitesse ou la capacité à s'hydrater ou à se solvater est supérieure en milieu aqueux exempt d'alcool qu'en solution alcoolique, l'agent D étant en mélange avec les microparticules de type réservoir et représentant de 2 à 30% p/p de la masse totale du mélange
ladite forme pharmaceutique orale résistant à la décharge immédiate de la dose de PA en présence d'alcool
ladite forme pharmaceutique ou diététique orale se présentant sous la forme d'une gélule,
ledit agent D étant choisi dans le groupe constitué par :
- les carboxyalkylcelluloses réticulées : les carboxyméthylcelluloses réticulées (e.g. croscarmellose de sodium),
- les (hydroxy)(alkyl)celluloses (e.g. hydroxypropylcellulose, hydroxypropyl-méthylcellulose),
- les carboxyalkylcelluloses (e.g. carboxyméthylcellulose) et leurs sels,
- les polysaccharides,
- les protéines,
- et leurs mélanges.

2. Forme pharmaceutique ou diététique orale selon la revendication 1, **caractérisée en ce que** le temps de libération de 50 % du PA, dans une solution alcoolique :
- n'est pas diminué de plus de 3 fois par rapport au temps de libération de 50 % du PA mesuré en milieu aqueux exempt d'alcool ;
- de préférence n'est pas diminué de plus de 2 fois par rapport au temps de libération de 50 % du PA mesuré en milieu aqueux exempt d'alcool ;
- de préférence n'est pas diminué de plus de 1,5 fois par rapport au temps de libération de 50 % du PA mesuré en milieu aqueux exempt d'alcool ;
- de préférence est similaire à celui mesuré en milieu aqueux exempt d'alcool, d'après le facteur de similarité f₂ ;
- voire le temps de libération de 50 % du PA en solution alcoolique est supérieur au temps de libération de 50 % du PA en milieu aqueux exempt d'alcool.

3. Forme pharmaceutique ou diététique orale selon la revendication 1, **caractérisée en ce qu'**elle comprend des microparticules de type réservoir :
- dont le diamètre moyen est inférieur à 2000 µm, plus préférentiellement compris entre 50 et 800 µm, et plus préférentiellement encore compris entre 100 et 600 µm.
- individuellement constituée par un coeur contenant le PA et enrobé par un enrobage comprenant :
• au moins un polymère insoluble A dans les liquides du tractus gastrointestinal ;
• au moins un agent plastifiant B ;
• éventuellement au moins un agent tensioactif C.

4. Forme pharmaceutique ou diététique orale selon la revendication 1, 2 ou 3, **caractérisée en ce que** l'agent D représente de 5% à 25% p/p, et plus préférentiellement encore de 5% à 20% p/p de la masse totale du mélange.

5. Forme pharmaceutique ou diététique orale selon la revendication 1, **caractérisée en ce que** l'agent D est choisi dans le groupe constitué par :
- les amidons natifs ou modifiés,
- les alginates et leurs sels tels que l'alginate de sodium,
- les gommes guar,
- les carraghénanes,
- les pullulanes,
- les pectines,
- les chitosanes et leurs dérivés
- la gélatine,
- les albumines,
- la caséine,
- les lactoglobulines,
- et leurs mélanges.

6. Forme pharmaceutique ou diététique orale selon revendication 5, **caractérisée en ce que** l'agent D est choisi dans le groupe constitué par :
- l'amidon de maïs, l'amidon de blé, l'amidon de pomme de terre
- l'amidon glycolate de sodium,
- et leurs mélanges.

7. Forme pharmaceutique ou diététique orale selon l'une quelconque des revendications 1 à 4, **caractérisée en ce que** l'agent D est choisi parmi :
- les hydroxyalkylcelluloses (e.g. hydroxypropylcellulose, hydroxypropyl-méthylcell ulose),
- les gommes guar,
- les carraghénanes,
- les pullulanes,
- et leurs mélanges.

8. Forme pharmaceutique ou diététique orale selon la revendication 3, **caractérisée en ce que** :
- le polymère A est présent dans l'enrobage des microparticules de type réservoir à raison de 70% à 95 % p/p de la masse totale de l'enrobage,
- l'agent plastifiant B est présent dans l'enrobage des microparticules de type réservoir à raison de 1 à 30 % p/p de la masse totale de l'enrobage,
- l'agent tensioactif C est présent dans l'enrobage des microparticules de type réservoir à raison de 0 à 30 % p/p de la masse totale de l'enrobage.

9. Forme pharmaceutique ou diététique orale selon la revendication 3, **caractérisée en ce que** le polymère A est sélectionné dans le groupe de produits suivants :
- les dérivés non hydrosolubles de la cellulose,
- les dérivés de (co)polymères (méth)acryliques,
- et leurs mélanges.

10. Forme pharmaceutique ou diététique orale selon la revendication 9, **caractérisée en ce que** le polymère A est sélectionné parmi : l'éthylcellulose, l'acétate butyrate de cellulose, l'acétate de cellulose, les copolymères ammonio-méthacrylates type A et type B (Eudragit® RS, Eudragit® RL, Eudragit® RSPO, Eudragit® PLPO), les esters d'acides poly(méth)acryliques (Eudragit® NE 30D) et leurs mélanges, l'éthylcellulose et/ou l'acétate de cellulose étant particulièrement préférés.

11. Forme pharmaceutique ou diététique orale selon la revendication 3, **caractérisée en ce que** l'agent plastifiant B est sélectionné parmi :
- le glycérol et ses esters, de préférence dans le sous-groupe suivant : glycérides acétylés, glycérolmono-stéarate, glycéryltriacétate, glycéroltributyrate,
- les phtalates, de préférence dans le sous-groupe suivant : dibutylphtalate, diéthylphtalate, diméthylphtalate, dioctylphtalate,
- les citrates, de préférence dans le sous-groupe suivant : acétyltributylcitrate, acétyltriéthylcitrate, tributylcitrate, triéthylcitrate,
- les sébacates, de préférence dans le sous-groupe suivant : diéthylsébacate, dibutylsébacate,
- les adipates,
- les azélates,
- les benzoates,
- le chlorobutanol,
- les polyéthylèneglycols,
- les huiles végétales,
- les fumarates, de préférence le diéthylfumarate,
- les malates, de préférence le diéthylmalate,
- les oxalates, de préférence le diéthyloxalate,
- les succinates ; de préférence le dibutylsuccinate,
- les butyrates,
- les esters de l'alcool cétylique,
- les malonates, de préférence le diéthylmalonate,
- l'huile de ricin,
- et leurs mélanges.

12. Forme pharmaceutique ou diététique orale selon la revendication 3, **caractérisée en ce qu'**elle comprend un agent tensioactif C sélectionné parmi :
- les sels alcalins ou alcalinoterreux des acides gras,
- les huiles polyoxyéthylénées,
- les copolymères polyoxyéthylène-polyoxypropylène,
- les esters de sorbitan polyoxyéthylénés,
- les dérivés de l'huile de ricin polyoxyéthylénés,
- les stéarates, de préférence de calcium, de magnésium, d'aluminium ou de zinc,
- les polysorbates,
- les stéarylfumarates, de préférence de sodium,
- le béhénate de glycérol,
- le chlorure de benzalkonium
- le bromure de cétyltriméthyl ammonium,
- et leurs mélanges.

13. Forme pharmaceutique ou diététique orale selon la revendication 3, **caractérisée en ce que** :
- le polymère A est l'éthylcellulose,
- l'agent plastifiant B est l'huile de ricin ;
- l'agent tensioactif C est le polysorbate ;
- l'agent D est choisi parmi la gomme guar, l'hydroxypropylméthylcellulose, l'hydroxypropylcellulose, la carboxy-méthylcellulose de sodium, le pullulane, le glycolate sodique d'amidon, et leurs mélanges.

14. Forme pharmaceutique ou diététique orale selon l'une quelconque des revendications 1 à 13, **caractérisée en ce qu'**elle contient des particules extrudées, les particules comprenant :
- des microparticules de type réservoir, à libération modifiée d'au moins un principe actif (PA) et
- au moins un agent D, l'agent D représentant de 5 à 20 % p/p des particules extrudées.

15. Forme pharmaceutique ou diététique orale selon l'une quelconque des revendications 1 à 14, **caractérisée en ce que** la gélule est à base de gélatine,
- revêtue d'un revêtement à base de carboxy-méthylcellulose de sodium, à raison de 25 % p/p de carboxy-méthylcellulose de sodium par rapport à la masse des gélules vides,
- contenant des microparticules de type réservoir.

16. Procédé pour l'obtention d'une forme pharmaceutique ou diététique orale selon l'une quelconque des revendications 1 à 15, en plusieurs étapes consistant essentiellement à :
a) préparer des coeurs (microparticules non enrobées) de PA par :
- extrusion/sphéronisation de PA avec éventuellement un ou plusieurs excipient(s) pharmaceutiquement acceptables, et/ou ;
- granulation humide de PA avec éventuellement un ou plusieurs excipient(s) pharmaceutiquement acceptables, et/ou ;
- compactage de PA avec éventuellement un ou plusieurs excipient(s) pharmaceutiquement acceptables, et/ou ;
- pulvérisation de PA, avec éventuellement un ou plusieurs excipient(s) pharmaceutiquement acceptables, en dispersion ou en solution dans un solvant aqueux ou organique sur un support neutre, et/ou ;
- tamisage de poudre ou cristaux de PA ;
b) préparer des microparticules de type réservoir de PA par :
- pulvérisation en lit d'air fluidisé d'une solution ou dispersion contenant un ou plusieurs composés A, B et éventuellement un ou plusieurs composés C sur les microparticules de PA ;
c) préparer la forme finale du médicament par :
- granulation et/ou extrusion/sphéronisation des microparticules de type réservoir de PA avec un agent D pour mise en gélule ; ou
- mise en gélule des microparticules de type réservoir de PA avec éventuellement un ou plusieurs agent(s) D et/ou des excipients pharmaceutiquement acceptables ; les gélules peuvent éventuellement être enrobées en turbine ou lit d'air fluidisé par un ou plusieurs agent(s) D et/ou des excipients pharmaceutiquement acceptables.

17. Forme pharmaceutique ou diététique orale se présentant sous la forme d'une gélule et comprenant des microparticules de type réservoir à libération prolongée d'au moins un PA et au moins un agent D qui est un composé pharmaceutiquement acceptable dont la vitesse ou la capacité à s'hydrater ou à se solvater est supérieure en milieu aqueux exempt d'alcool qu'en solution alcoolique, l'agent D étant dans un enrobage déposé sur la gélule,
ladite forme pharmaceutique orale résistant à la décharge immédiate de la dose de PA en présence d'alcool
ledit agent D étant choisi dans le groupe constitué par:
- les carboxyalkylcelluloses réticulées : les carboxyméthylcelluloses réticulées (e.g. croscarmellose de sodium),
- les (hydroxy)(alkyl)celluloses (e.g. hydroxypropylcellulose, hydroxypropyl-méthylcellulose),
- les carboxyalkylcelluloses (e.g. carboxyméthylcellulose) et leurs sels,
- les polysaccharides,
- les protéines, :
- et leurs mélanges.

18. Forme pharmaceutique ou diététique orale selon la revendication 17, **caractérisée en ce que** l'agent D est choisi dans le groupe constitué par :
- les amidons natifs ou modifiés,
- les alginates et leurs sels tels que l'alginate de sodium,
- les gommes guar,
- les carraghénanes,
- les pullulanes,
- les pectines,
- les chitosanes et leurs dérivés
- la gélatine,
- les albumines,
- la caséine,
- les lactoglobulines,
- et leurs mélanges.

19. Forme pharmaceutique ou diététique orale selon revendication 18, **caractérisée en ce que** l'agent D est choisi dans le groupe constitué par :
- l'amidon de maïs, l'amidon de blé, l'amidon de pomme de terre
- l'amidon glycolate de sodium,
- et leurs mélanges.

## Patentansprüche

1. Orale pharmazeutische oder diätetische Form mit Mikropartikeln vom Typ Reservoir mit verlängerter Freisetzung mindestens eines Wirkstoffs und mit mindestens einem Mittel D, bei dem es sich um eine pharmazeutisch zulässige Zusammensetzung handelt, deren Hydratationsgeschwindigkeit oder -fähigkeit bzw. deren Solvatisierungsgeschwindigkeit oder -fähigkeit in einem wässrigen Medium ohne Alkohol höher ist als in einer alkoholischen Lösung, wobei das Mittel D mit den Mikropartikeln vom Typ Reservoir vermischt ist und 2 bis 30 % w/w der Gesamtmasse des Gemischs bildet,
wobei die orale pharmazeutische Form gegenüber der unmittelbaren Freigabe der Wirkstoffdosis in Gegenwart von Alkohol beständig ist,
wobei die orale pharmazeutische oder diätetische Form in Form einer Kapsel vorliegt,
wobei das Mittel D aus der Gruppe ausgewählt ist, die besteht aus:
- vernetzten Carboxyalkylcellulosen: vernetzten Carboxymethylcellulosen (z.B. Croscarmellose-Natrium),
- (Hydroxy)(alkyl)cellulosen (z.B. Hydroxypropylcellulose, Hydroxypropylmethylcellulose),
- Carboxyalkylcellulosen (z.B. Carboxymethylcellulose) und ihren Salzen,
- Polysacchariden,
- Proteinen
- und aus ihren Gemischen.

2. Orale pharmazeutische oder diätetische Form nach Anspruch 1, **dadurch gekennzeichnet, dass** die Freisetzungsdauer von 50 % des Wirkstoffs in einer alkoholischen Lösung:
- bezüglich der in einem wässrigen Medium ohne Alkohol gemessenen Freisetzungsdauer von 50 % des Wirkstoffs um nicht mehr als das 3-fache verringert ist;
- bezüglich der in einem wässrigen Medium ohne Alkohol gemessenen Freisetzungsdauer von 50 % des Wirkstoffs vorzugsweise um nicht mehr als das 2-fache verringert ist;
- bezüglich der in einem wässrigen Medium ohne Alkohol gemessenen Freisetzungsdauer von 50 % des Wirkstoffs vorzugsweise um nicht mehr als das 1,5-fache verringert ist;
- vorzugsweise derjenigen, die in einem wässrigen Medium ohne Alkohol gemessenen wird, entsprechend dem Ähnlichkeitsfaktor f₂ ähnlich ist;
- wobei die Freisetzungsdauer von 50 % des Wirkstoffs in einer alkoholischen Lösung sogar länger ist als die Freisetzungsdauer von 50 % des Wirkstoffs in einem wässrigen Medium ohne Alkohol.

3. Orale pharmazeutische oder diätetische Form nach Anspruch 1, **dadurch gekennzeichnet, dass** sie Mikropartikel vom Typ Reservoir umfasst:
- deren mittlerer Durchmesser geringer ist als 2000 µm, vorzugsweise zwischen 50 und 800 µm und weiter vorzugsweise zwischen 100 und 600 µm beträgt,
- die individuell aus einem Kern bestehen, der den Wirkstoff enthält und mit einer Hülle umhüllt ist, die Folgendes enthält:
• mindestens ein Polymer A, das in den Flüssigkeiten des Magen-Darm-Trakts nicht löslich ist;
• mindestens einen Weichmacher B;
• gegebenenfalls mindestens ein oberflächenaktives Mittel C.

4. Orale pharmazeutische oder diätetische Form nach Anspruch 1, 2 oder 3, **dadurch gekennzeichnet, dass** das Mittel D 5 % bis 25 % w/w und weiter vorzugsweise 5 % bis 20 % w/w der Gesamtmasse des Gemischs bildet.

5. Orale pharmazeutische oder diätetische Form nach Anspruch 1, **dadurch gekennzeichnet, dass** das Mittel D aus der Gruppe ausgewählt ist, die besteht aus:
- natürlichen oder modifizierten Stärken,
- Alginaten und ihren Salzen, wie etwa Natriumalginat,
- Guargummis,
- Carrageenen,
- Pullulanen,
- Pektinen,
- Chitosanen und ihren Derivaten,
- Gelatine,
- Albuminen,
- Casein,
- Lactoglobulinen
- und aus ihren Gemischen.

6. Orale pharmazeutische oder diätetische Form nach Anspruch 5, **dadurch gekennzeichnet, dass** das Mittel D aus der Gruppe ausgewählt ist, die besteht aus:
- Maisstärke, Weizenstärke, Kartoffelstärke,
- Natriumglykolatstärke
- und aus ihren Gemischen.

7. Orale pharmazeutische oder diätetische Form nach einem der Ansprüche 1 bis 4, **dadurch gekennzeichnet, dass** das Mittel D ausgewählt ist aus:
- Hydroxyalkylcellulosen (z.B. Hydroxypropylcellulose, Hydroxypropylmethylcellulose),
- Guargummis,
- Carrageenen,
- Pullulanen
- und aus ihren Gemischen.

8. Orale pharmazeutische oder diätetische Form nach Anspruch 3, **dadurch gekennzeichnet, dass**:
- das Polymer A in einem Anteil von 70 % bis 95 % w/w der Gesamtmasse der Hülle in der Hülle der Mikropartikel vom Typ Reservoir vorhanden ist,
- der Weichmacher B in einem Anteil von 1 bis 30 % w/w der Gesamtmasse der Hülle in der Hülle der Mikropartikel vom Typ Reservoir vorhanden ist,
- das oberflächenaktive Mittel C in einem Anteil von 0 bis 30 % w/w der Gesamtmasse der Hülle in der Hülle der Mikropartikel vom Typ Reservoir vorhanden ist.

9. Orale pharmazeutische oder diätetische Form nach Anspruch 3, **dadurch gekennzeichnet, dass** das Polymer A aus der Gruppe der folgenden Produkte ausgewählt ist:
- nicht wasserlösliche Cellulosederivate,
- (Meth)acryl-(Co)polymerderivate
- und ihre Gemische.

10. Orale pharmazeutische oder diätetische Form nach Anspruch 9, **dadurch gekennzeichnet, dass** das Polymer A ausgewählt ist aus: Ethylcellulose, Celluloseacetatbutyrat, Celluloseacetat, Ammoniummethacrylat-Copolymeren Typ A und Typ B (Eudragit® RS, Eudragit® RL, Eudragit® RSPO, Eudragit® PLPO), Poly(meth)acrylsäureestern (Eudragit® NE 30D) und ihren Gemischen, wobei Ethylcellulose und/oder Celluloseacetat besonders bevorzugt sind.

11. Orale pharmazeutische oder diätetische Form nach Anspruch 3, **dadurch gekennzeichnet, dass** der Weichmacher B ausgewählt ist aus:
- Glycerin und seinen Estern, vorzugsweise in der folgenden Untergruppe: acetylierte Glyceride, Glycerolmonostearat, Glyceryltriacetat, Glyceroltributyrat,
- Phthalaten, vorzugsweise in der folgenden Untergruppe: Dibutylphthalat, Diethylphthalat, Dimethylphthalat, Dioctylphthalat,
- Citraten, vorzugsweise in der folgenden Untergruppe: Acetyltributylcitrat, Acetyltriethylcitrat, Tributylcitrat, Triethylcitrat,
- Sebacaten, vorzugsweise in der folgenden Untergruppe: Diethylsebacat, Dibutylsebacat,
- Adipaten,
- Azelaten,
- Benzoaten,
- Chlorbutanol,
- Polyethylenglycolen,
- Pflanzenölen,
- Fumaraten, vorzugsweise Diethylfumarat,
- Malaten, vorzugsweise Diethylmalat,
- Oxalaten, vorzugsweise Diethyloxalat,
- Succinaten, vorzugsweise Dibutylsuccinat,
- Butyraten,
- Cetylalkoholestern,
- Malonaten, vorzugsweise Diethylmalonat,
- Rizinusöl
- und aus ihren Gemischen.

12. Orale pharmazeutische oder diätetische Form nach Anspruch 3, **dadurch gekennzeichnet, dass** sie ein oberflächenaktives Mittel C enthält, das ausgewählt ist aus:
- Alkali- oder Erdalkalisalzen der Fettsäuren,
- polyoxyethylenierten Ölen,
- Polyoxyethylen-Polyoxypropylen-Copolymeren,
- polyoxyethylenierten Sorbitanestern,
- polyoxyethylenierten Rizinusölderivaten,
- Stearaten, vorzugsweise Calciumstearat, Magnesiumstearat, Aluminiumstearat oder Zinkstearat,
- Polysorbaten,
- Stearylfumaraten, vorzugsweise Natriumstearylfumarat,
- Glycerolbehenat,
- Benzalkoniumchlorid,
- Cetyltrimethylammoniumbromid
- und aus ihren Gemischen.

13. Orale pharmazeutische oder diätetische Form nach Anspruch 3, **dadurch gekennzeichnet, dass**:
- das Polymer A Ethylcellulose ist,
- der Weichmacher B Rizinusöl ist,
- das oberflächenaktive Mittel C Polysorbat ist,
- das Mittel D aus Guargummi, Hydroxypropylmethylcellulose, Hydroxypropylcellulose, Natriumcarboxymethylcellulose, Pullulan, Natriumglykolatstärke und ihren Gemischen ausgewählt ist.

14. Orale pharmazeutische oder diätetische Form nach einem der Ansprüche 1 bis 13, **dadurch gekennzeichnet, dass** sie extrudierte Partikel enthält, wobei die Partikel Folgendes umfassen:
- Mikropartikel vom Typ Reservoir mit modifizierter Freisetzung mindestens eines Wirkstoffs und
- mindestens ein Mittel D, wobei das Mittel D 5 bis 20 % w/w der extrudierten Partikel bildet.

15. Orale pharmazeutische oder diätetische Form nach einem der Ansprüche 1 bis 14, **dadurch gekennzeichnet, dass** die Kapsel auf Gelatinebasis ist,
- mit einem Überzug auf Natriumcarboxymethylcellulosebasis mit einem Natriumcarboxymethylcellulose-Anteil von 25 % w/w bezogen auf die Masse der leeren Kapseln überzogen ist,
- Mikropartikel vom Typ Reservoir enthält.

16. Verfahren zur Gewinnung einer oralen pharmazeutischen oder diätetischen Form nach einem der Ansprüche 1 bis 15 in mehreren Schritten, im Wesentlichen bestehend aus:
a) der Herstellung von Wirkstoffkernen (nicht umhüllten Mikropartikeln) durch:
- Wirkstoffextrusion/Wirkstoffsphäronisation gegebenenfalls mit einem oder mehreren pharmazeutisch zulässigen Arzneistoffträger(n) und/oder
- Wirkstofffeuchtgranulierung gegebenenfalls mit einem oder mehreren pharmazeutisch zulässigen Arzneistoffträger(n) und/oder
- Wirkstoffverdichtung gegebenenfalls mit einem oder mehreren pharmazeutisch zulässigen Arzneistoffträger(n) und/oder
- Wirkstoffzerstäubung gegebenenfalls mit einem oder mehreren pharmazeutisch zulässigen Arzneistoffträger(n) als Dispersion oder Lösung in einem wässrigen oder organischen Lösungsmittel auf einem neutralen Träger und/oder
- Sieben von Wirkstoffpulver oder -kristallen;
b) Herstellung von Wirkstoffmikropartikeln vom Typ Reservoir durch:
- Luftwirbelschichtzerstäubung einer Lösung oder Dispersion, die eine oder mehrere Zusammensetzungen A, B und gegebenenfalls eine oder mehrere Zusammensetzungen C enthält, auf den Wirkstoffmikropartikeln;
c) Herstellung der endgültigen Form des Arzneimittels durch:
- Granulierung und/oder Extrusion/Sphäronisation der Wirkstoffmikropartikel vom Typ Reservoir mit einem Mittel D für die Verkapselung; oder
- Verkapselung der Wirkstoffmikropartikel vom Typ Reservoir gegebenenfalls mit einem oder mehreren Mittel(n) D und/oder pharmazeutisch zulässigen Arzneistoffträger(n), wobei die Kapseln gegebenenfalls mit einem oder mehreren Mittel(n) D und/oder pharmazeutisch zulässigen Arzneistoffträger(n) in einer Turbine oder durch Luftwirbelschicht umhüllt werden können.

17. Orale pharmazeutische oder diätetische Form, die in Form einer Kapsel vorliegt und die Mikropartikel vom Typ Reservoir mit verlängerter Freisetzung mindestens eines Wirkstoffs und mindestens ein Mittel D umfasst, bei dem es sich um eine pharmazeutisch zulässige Zusammensetzung handelt, deren Hydratationsgeschwindigkeit oder -fähigkeit bzw. deren Solvatisierungsgeschwindigkeit oder -fähigkeit in einem wässrigen Medium ohne Alkohol höher ist als in einer alkoholischen Lösung, wobei sich das Mittel D in einer auf der Kapsel aufgebrachten Hülle befindet,
wobei die orale pharmazeutische Form gegenüber der unmittelbaren Freigabe der Wirkstoffdosis in Gegenwart von Alkohol beständig ist,
wobei das Mittel D aus der Gruppe ausgewählt ist, die besteht aus:
- vernetzten Carboxyalkylcellulosen: vernetzten Carboxymethylcellulosen (z.B. Croscarmellose-Natrium),
- (Hydroxy)(alkyl)cellulosen (z.B. Hydroxypropylcellulose, Hydroxypropylmethylcellulose),
- Carboxyalkylcellulosen (z.B. Carboxymethylcellulose) und ihren Salzen,
- Polysacchariden,
- Proteinen
- und aus ihren Gemischen.

18. Orale pharmazeutische oder diätetische Form nach Anspruch 17, **dadurch gekennzeichnet, dass** das Mittel D aus der Gruppe ausgewählt ist, die besteht aus:
- natürlichen oder modifizierten Stärken,
- Alginaten und ihren Salzen, wie etwa Natriumalginat,
- Guargummis,
- Carrageenen,
- Pullulanen,
- Pektinen,
- Chitosanen und ihren Derivaten,
- Gelatine,
- Albuminen,
- Casein,
- Lactoglobulinen
- und aus ihren Gemischen.

19. Orale pharmazeutische oder diätetische Form nach Anspruch 18, **dadurch gekennzeichnet, dass** das Mittel D aus der Gruppe ausgewählt ist, die besteht aus:
- Maisstärke, Weizenstärke, Kartoffelstärke,
- Natriumglykolatstärke
- und aus ihren Gemischen.

## Claims

1. Oral pharmaceutical or dietary form comprising reservoir-type microparticles with extended release of at least one active principle (AP) and at least one agent D which is a pharmaceutically acceptable compound of which the hydrating or solvating speed and capacity is higher in an alcohol-free aqueous medium than in an alcohol-containing solution, the agent D being in a mixture with the reservoir-type microparticles and representing from 2 to 30% w/w of the total mass of the mixture said oral pharmaceutical form resistant to the immediate release of the dose of AP in the presence of alcohol
said oral pharmaceutical or dietary form having the form of a capsule,
said agent D being chosen in the group comprising:
- cross-linked carboxyalkylcelluloses: cross-linked carboxymethylcelluloses (e.g. croscarmellose sodium),
- (hydroxy)(alkyl)celluloses (e.g. hydroxypropylcellulose, hydroxypropyllmethylcellulose),
- carboxyalkylcelluloses (e.g. carboxymethylcellulose) and the salts thereof,
- polysaccharides,
- proteins,
- and mixtures thereof.

2. Oral pharmaceutical or dietary form according to claim 1, **characterised in that** the releasing time of 50% of the AP, in an alcohol-containing solution:
- is not reduced by more than 3 times compared to the releasing time of 50% of the AP measured in an alcohol-free aqueous medium;
- preferably is not reduced by more than 2 times compared to the releasing time of 50% of the AP measured in an alcohol-free aqueous medium;
- preferably is not reduced by more than 1.5 times compared to the releasing time of 50% of the AP measured in an alcohol-free aqueous medium;
- preferably is similar to that measured in an alcohol-free aqueous medium, according to the similarity factor f₂;
- even the releasing time of 50% of the AP in an alcohol-containing solution is greater than the releasing time of 50% of the AP in an alcohol-free aqueous medium.

3. Oral pharmaceutical or dietary form according to claim 1, **characterised in that** it comprises reservoir-type microparticles:
- of which the average diameter is less than 2000 µm, more preferably between 50 and 800 µm, and even more preferably between 100 and 600 µm.
- individually comprised of a core containing the AP and coated by a coating comprising:
• at least one insoluble polymer A in the liquids of the gastrointestinal tract;
• at least one plasticizing agent B;
• optionally at least one surfactant C.

4. Oral pharmaceutical or dietary form according to claim 1, 2 or 3, **characterised in that** the agent D represents from 5% to 25% w/w, and even more preferably from 5% to 20% w/w of the total mass of the mixture.

5. Oral pharmaceutical or dietary form according to claim 1, **characterised in that** the agent D is chosen in the group comprising:
- native or modified starches,
- alginates and the salts thereof such as sodium alginate,
- guar gums,
- carrageenans,
- pullulans,
- pectins,
- chitosans and the derivatives thereof
- gelatine,
- albumins,
- casein,
- lactoglobulins,
- and mixtures thereof.

6. Oral pharmaceutical or dietary form according to claim 5, **characterised in that** the agent D is chosen in the group comprising:
- corn starch, wheat starch, potato starch
- sodium starch glycolate,
- and mixtures thereof.

7. Oral pharmaceutical or dietary form according to any of claims 1 to 4, **characterised in that** the agent D is chosen from:
- hydroxyalkylcelluloses (e.g. hydroxypropylcellulose, hydroxypropyllmethylcellulose),
- guar gums,
- carrageenans,
- pullulans,
- and mixtures thereof.

8. Oral pharmaceutical or dietary form according to claim 3, **characterised in that**:
- the polymer A is present in the coating of the reservoir-type microparticles at a rate of 70% to 95% w/w of the total mass of the coating,
- the plasticizing agent B is present in the coating of the reservoir-type microparticles at a rate of 1 to 30% w/w of the total mass of the coating,
- the surfactant C is present in the coating of the reservoir-type microparticles at a rate of 0 to 30% w/w of the total mass of the coating.

9. Oral pharmaceutical or dietary form according to claim 3, **characterised in that** the polymer A is selected in the following group of products:
- water-insoluble derivatives of cellulose,
- derivatives of (meth)acrylic (co)polymers,
- and mixtures thereof.

10. Oral pharmaceutical or dietary form according to claim 9, **characterised in that** the polymer A is selected from: ethylcellulose, cellulose acetate butyrate, cellulose acetate, type A and type B ammonio methacrylate copolymers (Eudragit® RS, Eudragit® RL, Eudragit® RSPO, Eudragit® PLPO), esters of poly(meth)acrylic acids (Eudragit® NE 30D) and mixtures thereof, ethylcellulose and/or cellulose acetate being particularly preferred.

11. Oral pharmaceutical or dietary form according to claim 3, **characterised in that** the plasticizing agent B is selected from:
- glycerol and the esters thereof, preferably in the following sub-group: acetylated glycerides, glycerolmono-stearate, glyceryltriacetate, glyceroltributyrate,
- phthalates, preferably in the following sub-group: dibutylphthalate, diethylphthalate, dimethylphthalate, dioctylphthalate,
- citrates, preferably in the following sub-group: acetyltributylcitrate, acetyltriethylcitrate, tributylcitrate, triethylcitrate,
- sebacates, preferably in the following sub-group: diethylsebacate, dibutylsebacate,
- adipates,
- azelates,
- benzoates,
- chlorobutanol,
- polyethyleneglycols,
- plant oils,
- fumarates, preferably diethylfumarate,
- malates, preferably diethylmalate,
- oxalates, preferably diethyloxalate,
- succinates; preferably dibutylsuccinate,
- butyrates,
- cetyl alcohol esters,
- malonates, preferably diethylmalonate,
- castor oil,
- and mixtures thereof.

12. Oral pharmaceutical or dietary form according to claim 3,
**characterised in that** it comprises a surfactant C selected from:
- alkaline or alkaline earth salts of fatty acids,
- polyoxyethylenated oils,
- polyoxyethylene-polyoxypropylene copolymers,
- polyoxyethylene sorbitan esters,
- derivatives of polyoxyethylenated castor oil,
- stearates, preferably of calcium, magnesium, aluminium or zinc,
- polysorbates,
- stearylfumarates, preferably of sodium,
- glyceryl behenate,
- benzalkonium chloride
- cetyltrimethyl ammonium bromide,
- and mixtures thereof.

13. Oral pharmaceutical or dietary form according to claim 3, **characterised in that**:
- the polymer A is ethylcellulose,
- the plasticizing agent B is castor oil;
- the surfactant C is polysorbate;
- the agent D is chosen from guar gum, hydroxypropylmethylcellulose, hydroxypropylcellulose, sodium carboxy-methylcellulose, pullulan, sodium starch glycolate, and mixtures thereof.

14. Oral pharmaceutical or dietary form according to any of claims 1 to 13, **characterised in that** it contains extruded particles, with the particles comprising:
- reservoir-type microparticles, with modified release of at least one active principle (AP) and
- at least one agent D, the agent D representing from 5 to 20% w/w of the extruded particles.

15. Oral pharmaceutical or dietary form according to any of claims 1 to 14, **characterised in that** the capsule has a gelatine base,
- coated with a sodium carboxy-methylcellulose coating, at a rate of 25% w/w of sodium carboxy-methylcellulose with respect to the weight of the empty capsules,
- containing reservoir-type microparticles.

16. Method for obtaining an oral pharmaceutical or dietary form according to any of claims 1 to 15, in several steps consisting substantially in:
a) preparing cores (uncoated microparticles) of AP by:
- extrusion/spheronisation of AP with optionally one or several pharmaceutically acceptable excipients, and/or;
- wet granulation of AP with optionally one or several pharmaceutically acceptable excipients, and/or;
- compaction of AP with optionally one or several pharmaceutically acceptable excipients, and/or;
- spraying of AP, with optionally one or several pharmaceutically acceptable excipients, in a dispersion or in a solution in an aqueous or organic solvent on a neutral support, and/or;
- screening of powder or crystals of AP;
b) preparing reservoir-type microparticles of AP by:
- spraying in a fluidised bed of a solution or dispersion containing one or several compounds A, B and optionally one or several compounds C on the microparticles of AP;
c) preparing the final form of the drug by:
- granulation and/or extrusion/spheronisation of the reservoir-type microparticles of AP with an agent D for capsule filling; or
- capsule filling of reservoir-type microparticles of AP with optionally one or several agent(s) D and/or pharmaceutically acceptable excipients; the capsules can optionally be coated with turbine or fluidised bed by one or several agent(s) D and/or pharmaceutically acceptable excipients.

17. Oral pharmaceutical or dietary form having the form of a capsule and comprising reservoir-type microparticles with extended release of at least one AP and at least one agent D which is a pharmaceutically acceptable compound of which the hydrating or solvating speed and capacity is higher in an alcohol-free aqueous medium than in an alcohol-containing solution, the agent D being in a coating deposited on the capsule,
said oral pharmaceutical form resistant to the immediate release of the dose of AP in the presence of alcohol
said agent D being chosen in the group comprising:
- cross-linked carboxyalkylcelluloses: cross-linked carboxymethylcelluloses (e.g. croscarmellose sodium),
- (hydroxy)(alkyl)celluloses (e.g. hydroxypropylcellulose, hydroxypropyllmethylcellulose),
- carboxyalkylcelluloses (e.g. carboxymethylcellulose) and the salts thereof,
- polysaccharides,
- proteins
- and mixtures thereof.

18. Oral pharmaceutical or dietary form according to claim 17, **characterised in that** the agent D is chosen in the group comprising:
- native or modified starches,
- alginates and the salts thereof such as sodium alginate,
- guar gums,
- carrageenans,
- pullulans,
- pectins,
- chitosans and the derivatives thereof
- gelatine,
- albumins,
- casein,
- lactoglobulins,
- and mixtures thereof.

19. Oral pharmaceutical or dietary form according to claim 18, **characterised in that** the agent D is chosen in the group comprising:
- corn starch, wheat starch, potato starch
- sodium starch glycolate,
- and mixtures thereof.
